# EUROPEAN PATENT SPECIFICATION

(11) **EP 4 011 453 B1**
(45) Date of publication and mention of the grant of the patent: **25.11.2026**
(21) Application number: 20853185.5
(22) Date of filing: 07.08.2020
(51) Int. Cl.: A61P 35/00, C12N 7/04, C12N 15/12, C12N 15/39, C12N 15/863, A61K 35/76

(54) **ONCOLYTIC VACCINIA VIRUS**
ONKOLYTISCHES VACCINIA-VIRUS
VIRUS DE LA VACCINE ONCOLYTIQUE

(30) Priority: 09.08.2019 JP 2019147885
(43) Date of publication of application: 15.06.2022
(73) Proprietor: KM Biologics Co., Ltd., Kumamoto-shi, Kumamoto 860-8568 (JP)
(72) Inventor: MURAKAMI, Toshio, Kikuchi-shi, Kumamoto 869-1298 (JP); OKITA, Go, Kikuchi-shi, Kumamoto 869-1298 (JP); KAMIZURU, Yui, Kikuchi-shi, Kumamoto 869-1298 (JP)
(74) Representative: Vossius & Partner Patentanwälte Rechtsanwälte mbB
(86) International application number: PCT/JP2020/030448
(87) International publication number: WO 2021/029385

(56) References cited:
- WO-A1-2015/076422
- JP-A- 2006 506 974
- JP-A- 2011 504 104
- JP-A- 2016 178 945
- JP-A- 2017 524 693
- GOEBEL S J ET AL: "THE COMPLETE DNA SEQUENCE OF VACCINIA VIRUS", VIROLOGY, ELSEVIER, AMSTERDAM, NL, vol. 179, no. 1, 1 November 1990 (1990-11-01), pages 247 - 266, XP000253545, ISSN: 0042-6822, DOI: 10.1016/0042-6822(90)90294-2
- MCINTOSH A A G & SMITH G L: "Vaccinia virus glycoprotein A34R is required for infectivity of extracellular enveloped virus", JOURNAL OF VIROLOGY, THE AMERICAN SOCIETY FOR MICROBIOLOGY, US, vol. 70, no. 1, 1 January 1996 (1996-01-01), pages 272 - 281, XP002409051, ISSN: 0022-538X
- POTTS KYLE G ET AL: "Deletion of F4L (ribonucleotide reductase) in vaccinia virus produces a selective oncolytic virus and promotes anti‐tumor immunity with superior safety in bladder cancer models", EMBO MOLECULAR MEDICINE, vol. 9, no. 5, 1 May 2017 (2017-05-01), US, pages 638 - 654, XP055793215, ISSN: 1757-4676, DOI: 10.15252/emmm.201607296
- MCCART A ET AL: "Systemic Cancer Therapy with a Tumor-selective Vaccinia Virus Mutant Lacking Thymidine Kinase and Vaccinia Growth Factor Genes", CANCER RESEARCH, AMERICAN ASSOCIATION FOR CANCER RESEARCH, US, vol. 61, no. 24, 25 December 2001 (2001-12-25), pages 8751 - 8757, XP008121980, ISSN: 0008-5472
- MOTOMU NAKATAKE ET AL: "Partial Deletion of Glycoprotein B5R Enhances Vaccinia Virus Neutralization Escape while Preserving Oncolytic Function", MOLECULAR THERAPY - ONCOLYTICS, vol. 14, 1 September 2019 (2019-09-01), pages 159 - 171, XP055656662, ISSN: 2372-7705, DOI: 10.1016/j.omto.2019.05.003
- POTTS, KYLE G ET AL.: "Deletion of F4L (ribonucleotide reductase) in vaccinia virus produces a selective oncolytic virus and promotes anti-tumor immunity with superior safety in bladder cancer models", EMBO MOLECULAR MEDICINE, vol. 9, no. 5, May 2017 (2017-05-01), pages 638 - 654, XP055793215

## Description

### TECHNICAL FIELD

The present description generally relates to an oncolytic vaccinia virus that can be intravenously administered and has the features of improving safety by restricting a virus replication in normal cells by impairing the functions of vaccinia virus growth factor (VGF), extracellular signal-regulated kinase (ERK)-activating protein, and ribonucleotide reductase (RNR), and improving virus production in tumor cells and in host cells used for industrial production by changing an amino acid residue(s) of a protein(s) involving formation of virions of extracellular enveloped virus (EEV) of vaccinia virus. The present invention relates to a vaccinia virus deficient in functions of vaccinia virus growth factor (VGF), extracellular signal-regulated kinase (ERK)-activating protein, and ribonucleotide reductase (RNR), wherein all or part of regions of each of C11R, O1L, and F4L genes have been deleted or modified, and the functions of these gene products have been inactivated. The present invention also relates to a pharmaceutical composition for use in treating a cancer comprising said virus and to a growth-restricted vaccinia virus vector, which is said virus into which a foreign DNA has been introduced. The present invention also relates to a method of improving productivity of a growth-restricted vaccinia virus comprising replacing a DNA sequence(s) of a gene(s) encoding an extracellular enveloped virus (EEV)-related protein(s) of the growth-restricted vaccinia virus by a DNA sequence(s) of a corresponding gene(s) of another vaccinia virus strain having a high EEV productivity, wherein the growth-restricted vaccinia virus does not replicate in a normal cell, and can selectively replicate in a proliferating cell.
The vaccinia virus of the present invention has improved virus safety and productivity, and can be effectively used as an anticancer agent.

### BACKGROUND ART

A Mitogen-activated protein kinase-Dependent Recombinant Vaccinia Virus (MD-RVV) has been disclosed as an oncolytic vaccinia virus in which both genes encoding vaccinia virus growth factor (hereinafter, also referred to as "VGF") and O1L protein, which is a protein that activates extracellular signal-regulated kinase (hereinafter, also referred to as "ERK") are deleted to restrict growth in normal cells, however specifically proliferate in cancer cells, thereby damaging the cancer cells (Patent Document 1).

The vaccinia virus utilizes the epidermal growth factor (hereinafter, also referred to as "EGF") receptor signaling pathway to promote the spread of the virus through rapid and direct motility of infected cells (Non-Patent Document 1). C11R protein, a vaccinia virus growth factor (VGF) that is highly homologous to EGF and is secreted at an early stage of vaccinia virus infection, binds to an EGF receptor on infected cells and surrounding cells to transduce a signal through the MAP kinase cascade (Ras/Raf/MEK/ERK metabolic pathway). In addition, the O1L protein of vaccinia virus constitutively activates extracellular signal-regulated kinase (ERK) in infected cells and promotes the pathogenicity of the virus (Non-Patent Document 2). The C11R and O1L deficient virus (MD-RVV) lowers viral growth because ERK cannot be activated in normal cells. In cancer cells with abnormally activated ERK pathways, however, the inactivated virus ERK activation function is complemented, and the virus proliferates and becomes an oncolytic virus that destroys the cancer cells (Patent Document 1).

In order to reduce virus toxicity, disclosed is an oncolytic virus using a poxvirus in which the ribonucleotide reductase encoded in the viral genome is inactivated (Patent Document 2).

It is well known that a point mutation at amino acid position K151 of the A34R polypeptide of vaccinia virus improves the production of extracellular enveloped virus (Non-Patent Document 3 and Patent Document 3).

### PRIOR ART DOCUMENTS

### PATENT DOCUMENTS

Patent Document 1: JP-A-2015-076422
Patent Document 2: JP-A-2011-504104
Patent Document 3: JP-A-2006-506974

### NON-PATENT DOCUMENTS

Non-Patent Document 1: Beerli C., et al., Nature Microbiology, 4(2), 216-225, 2019
Non-Patent Document 2: Schweneker, M., et al., Journal of Virology, 86(4), 2323-2336, 2012
Non-Patent Document 3: Blasco, R., et al., Journal of Virology, 67(6), 3319-3325, 1993
Non-Patent Document 4: Downs-Canner, S., et al., Molecular Therapy, 24(8), 1492-1501, 2016
Non-Patent Document 5: Loren, K., et al., Clinical Cancer Research, 23(19), 5696-5702, 2017
Non-Patent Document 6: McIntosh A. A., Smith G. L., Journal of Virology, 70(1), 272-281, 1996
Non-Patent Document 7: Ferguson M. S., et al., Advances in Virology, 2012, 805629, 2012
Non-Patent Document 8: Badrinath N, et al., International Journal of Nanomedicine, 11, 4835-4847, 2016
Non-Patent Document 9: Bernet J., et al., Journal of Biosciences, 28(3), 249-264, 2003
Non-Patent Document 10: Dehaven B. C., et al., Journal of General Virology, 92, 1971-1980, 2011
Non-Patent Document 11: Chung, C.-S., Journal of Virology, 72(2), 1577-1585, 1998
Non-Patent Document 12: Gammon, D. B., et al., PLOS Pathogens, 6(7), e1000984, 2010
Non-Patent Document 13: Aye Y., et al., Oncogene, 34(16), 2011-2021, 2015
Non-Patent Document 14: Engstroem Y., et al., The Journal of Biological Chemistry, 260(16), 9114-9116, 1985
Non-Patent Document 15: Torii, S., et al., Cancer Science, 97(8), 697-702, 2006

### SUMMARY OF THE INVENTION

The invention is set out in the appended set of claims.

### PROBLEMS TO BE SOLVED BY THE INVENTION

Oncolytic vaccinia viruses are expected to be effective not only for treatment of a primary tumor by intratumoral administration but also for systemic micrometastasis by systemic administration using an intravenous injection. However, in a previous clinical trial in which an oncolytic vaccinia virus was administered intravenously, adverse effects associated with virus excretion were observed along with results suggesting efficacy (Non-Patent Document 4; Non-Patent Document 5). Further improvement in the safety of the oncolytic virus has been sought.

On the other hand, oncolytic viruses that have been genetically modified to suppress the virus proliferation in normal cells to improve safety are different from their parent strain virus before genetic modification in cancer cells and host cells used for industrial production. In comparison, it is often accompanied by a decrease in virus proliferation, and there is a concern about a decrease in efficacy and productivity. Thus, it has been desired to improve the safety of the virus as well as the productivity.

However, for improved efficacy and productivity, it is useful to modify the gene(s) encoding the extracellular enveloped virus (EEV)-related protein(s) to increase the proportion of EEV. However, the increase in EEV production involves virus toxicity (Non-Patent Document 6).

Previously reported clinical trial data about intravenous administration of oncolytic viruses have shown that the viruses can be safely and systemically delivered with limited toxicity, however there are individual differences in efficacy. Such incompleteness has been found. This is mainly because at the dose and dosage regimen that have been confirmed to be safe, the virus is rapidly cleared from the circulation before it reaches its target. This phenomenon occurs primarily due to neutralizing antibodies, complement activation, antiviral cytokines, and endogenous tissue macrophages, with nonspecific uptake by other tissues such as the lung, liver and spleen (Non-Patent Document 7). Among the oncolytic viruses, vaccinia virus has become a focus of preclinical and clinical studies due to its many favorable properties (Non-Patent Document 8). Some oncolytic vaccinia viruses have been clinically tested by intravenous administration. The vaccinia virus complement regulatory protein secreted by vaccinia virus (hereinafter also referred to as "VCP") binds and inactivates complements C4b and C3b, thereby inhibiting the classical and alternative pathways of complement activation (Non-Patent Document 9). In addition, the vaccinia virus in the EEV form incorporates a host protein in its membrane, which protein can prevent complement activation, and the EEV protein A56R also fixes the secreted VCP to protect it from complement attacks (Non-Patent Document 10).

The vaccinia virus does not have a specific receptor. Here, glycosaminoglycans, such as heparan sulfate, which are ubiquitously present in all tissues primarily including a connective tissue of animals, mediate the interaction between the vaccinia virus and host cells. The virus fuses directly with the plasma membrane and enters a tumor cell by endocytosis (Non-Patent Document 11). Thus, oncolytic vaccinia viruses do not exhibit cell preference specific to the organ, and should have a therapeutic effect on a wide range of cancer types. On the other hand, upon treatment by intravenous administration, systemic side effects due to viral growth become a problem. In order to solve this problem, it is necessary to strictly restrict the intracellular viral replication that occurs after the vaccinia virus infects normal cells and invades them. In general, since the effective range and the toxic range of each anticancer drug are close to each other, adverse effects occur at the dose and dosage regimen expected to be effective.

### SOLUTIONS TO THE PROBLEMS

The vaccinia virus genome has a gene encoding ribonucleotide reductase (hereinafter also referred to as "RNR"), which is a rate-limiting enzyme in DNA synthesis and includes I4L (large subunit; RRM1) and F4L (small subunit; RRM2). F4L is required for efficient replication in cultured cells and virus toxicity in mice (Non-Patent Document 12). Meanwhile, elevated RNR expression in cells is characteristic of many cancers, and as a result of investigating RNR gene expression in human cancers by using the ONCOMINE database, RRM2 was ranked in top 10% among genes most overexpressed in 73 of 168 cancers analyzed (Non-Patent Document 13). In addition, RNR activity in mammals is cell cycle-dependent, and the protein level of RRM1 are constant throughout the cell cycle, while RRM2 is expressed in the G1/S phase during DNA replication, and cell cycle-dependent enzyme activity is regulated by the level of RRM2 (Non-Patent Document 14). By contrast, the progression of the cell cycle from G1 phase to S phase is triggered by the activated ERK pathway (Non-Patent Document 15). Accordingly, the vaccinia virus with inhibited F4L functions can replicate using cell-derived RRM2 in cancer cells where the cells are actively proliferating. In normal cells, however, the expression level of RRM2 is low, so that viral replication is restricted. Further deletion of F4L from MD-RVV in which the functions of C11R and O1L are inhibited significantly suppresses viral growth in normal cells and further improves safety.

It is predicted that oncolytic viruses, which are growth-restricted viruses that cannot proliferate in normal cells and can selectively grow in target cancer cells, tend to have a less growth potential than parental viruses even in tumor cells and host cells used for industrial production. The oncolytic vaccinia virus, from which the F4L gene as well as the above-mentioned C11R and O1L genes have been deleted, complements ERK and RNR which are required for viral replication, by using enzymes in the highly replicative cancer cells, so that the virus can replicate.

As a method of further complementing productivity of a virus that lacks the functions of ERK and RNR related to cell proliferation, the present inventors have focused on a virion formation-related molecule(s) expressed during the late phase of virus life cycle while complementing the ERK and RNR activities by using enzymes derived from cancer cells. Here, there are four different forms of virion of vaccinia virus (including intracellular mature virus (hereinafter, also referred to as "IMV"), intracellular enveloped virus (hereinafter, also referred to as "IEV"), cell-associated enveloped virus (hereinafter, also referred to as "CEV"), and extracellular enveloped virus (hereinafter, also referred to as "EEV")). The genes encoding the seven different proteins (A33R, A34R, A36R, A56R, B5R, F12L, and F13L) that are components of EEV may be replaced with the corresponding genes of another vaccinia virus strain having a high EEV productivity. This has been found to improve viral productivity and make intravenous administration safe. Then, the present invention has been completed.

The present invention provides the following [1] to [20].
[1] A vaccinia virus deficient in functions of vaccinia virus growth factor (VGF), extracellular signal-regulated kinase (ERK)-activating protein, and ribonucleotide reductase (RNR), wherein all or part of regions of each of C11R, O1L, and F4L genes have been deleted or modified, and the functions of these gene products have been inactivated.
[2] The virus according to [1], which is a growth-restricted virus having improved safety, wherein the virus does not replicate in a normal cell and can selectively replicate in a proliferating cell.
[3] The virus according to [1], wherein the virus is a growth-restricted vaccinia virus, and a gene(s) encoding extracellular enveloped virus (EEV)-related protein(s) is replaced by a corresponding gene(s) of another vaccinia virus strain having a high EEV productivity, and wherein the virus does not replicate in a normal cell and can selectively replicate in a proliferating cell, thereby having improved safety and productivity.
[4] The virus according to [3], wherein the gene(s) encoding the extracellular enveloped virus (EEV)-related protein(s)
   (i) is one or more genes selected from the group consisting of A33R, A34R, A36R, A56R, B5R, F12L, and F13L, and has been replaced by a corresponding gene(s) of another vaccinia virus strain having a high EEV productivity;
   (ii) are A33R, A36R, A56R, B5R, F12L, and F13L, and have been replaced by corresponding genes of another vaccinia virus strain having a high EEV productivity; or
   (iii)are A33R, A34R, A36R, A56R, B5R, F12L, and F13L, and have been replaced by corresponding genes of another vaccinia virus strain having a high EEV productivity.
[5] The virus according to [3] or [4], wherein another vaccinia virus strain having a high EEV productivity is IHD-J strain or IHD-W strain.
[6] A pharmaceutical composition for use in treating a cancer, comprising the virus according to any one of [1] to [5].
[7] The pharmaceutical composition for use according to [6], which is for intravenous administration, intraperitoneal administration, or intratumoral administration.
[8] A growth-restricted vaccinia virus vector, which is the virus according to any one of [1] to [5] into which a foreign DNA has been introduced.
[9] The vector according to [8], wherein the foreign DNA is a gene encoding a cancer-specific antigen, an immune response regulator, or a protein with affinity for a cancer cell surface antigen.
[10] The virus according to [1], wherein the virus is a growth-restricted vaccinia virus having improved productivity, and a DNA sequence(s) of a gene(s) encoding extracellular enveloped virus (EEV)-related protein(s) of the growth-restricted vaccinia virus has been replaced by a DNA sequence(s) of a corresponding gene(s) of another vaccinia virus strain having a high EEV productivity, and wherein the virus does not replicate in a normal cell, and can selectively replicate in a proliferating cell.
[11] The virus according to [10], wherein the gene(s) encoding the extracellular enveloped virus (EEV)-related protein(s)
   (i) is one or more genes selected from the group consisting of A33R, A34R, A36R, A56R, B5R, F12L, and F13L;
   (ii) are A33R, A36R, A56R, B5R, F12L, and F13L genes; or
   (iii) are A33R, A34R, A36R, A56R, B5R, F12L, and F13L genes.
[12] The virus according to [10] or [11], wherein another vaccinia virus strain having a high EEV productivity is IHD-J strain or IHD-W strain.
[13] A pharmaceutical composition for use in treating a cancer, comprising the virus according to any one of [10] to [12].
[14] A growth-restricted vaccinia virus vector, which is the virus according to any one of [10] to [12] into which a foreign DNA has been introduced.
[15] The vector according to [14], wherein the foreign DNA is a gene encoding a cancer-specific antigen, an immune response regulator, or a protein with affinity for a cancer cell surface antigen.
[16] A method of improving productivity of a growth-restricted vaccinia virus, comprising:
   replacing a DNA sequence(s) of a gene(s) encoding an extracellular enveloped virus (EEV)-related protein(s) of the growth-restricted vaccinia virus by a DNA sequence(s) of a corresponding gene(s) of another vaccinia virus strain having a high EEV productivity, wherein the growth-restricted vaccinia virus does not replicate in a normal cell, and can selectively replicate in a proliferating cell.
[17] The method according to [16], wherein the gene(s) encoding the extracellular enveloped virus (EEV)-related protein(s)
   (i) is one or more genes selected from the group consisting of A33R, A34R, A36R, A56R, B5R, F12L, and F13L;
   (ii) are A33R, A36R, A56R, B5R, F12L, and F13L genes; or
   (iii)are A33R, A34R, A36R, A56R, B5R, F12L, and F13L genes.
[18] The method according to [16], wherein another vaccinia virus strain having a high EEV productivity is IHD-J strain or IHD-W strain.

The present invention is further described in the following .

### EFFECTS OF THE INVENTION

The present invention can provide an intravenously administrable oncolytic vaccinia virus with improved productivity by impairing the functions of vaccinia virus-derived VGF, ERK and RNR so as to remarkably suppress viral growth in normal cells, thereby improving safety, and by replacing a gene(s) encoding an EEV-related protein(s) of the virus by a corresponding gene(s) of another vaccinia virus strain with a high EEV productivity. The virus can be effectively used as a novel anticancer agent.

### BRIEF DESCRIPTION OF THE DRAWINGS

Fig. 1 is a diagram showing the structure of each exemplary recombinant vaccinia virus of the present invention.
Fig. 2 is a diagram illustrating how to produce an exemplary recombinant vaccinia virus of the present invention.
Fig. 3 is a graph showing the effects of F4L-deficient modified virus on the cytotoxicity in cancer cells or normal cells. A: HeLa (human cervical carcinoma cells); B: NHDF (normal human dermal fibroblasts).
Fig. 4 is a chart showing the survival rates of immunodeficient mice intravenously administered with F4L-deficient modified viruses.
Fig. 5 is a chart showing body weight changes in immunodeficient mice intravenously administered with F4L-deficient modified viruses.
Fig. 6 is a chart showing changes in the state of immunodeficient mice intravenously administered with F4L-deficient modified viruses.
Fig. 7 is a graph showing the effects of modification of EEV-related proteins on productivity in HeLa cells using a serum-containing medium. Modification is based on (A) MD-RVV and (B) a virus lacking the F4L gene of MD-RVV.
Fig. 8 is a graph showing the effects of modification of EEV-related proteins on productivity in HeLa cells using a chemically-defined culture medium. A: Culture supernatant virus; B: Intracellular virus.
Fig. 9 is a chart showing viral symptom scores of peritoneally metastasized human pancreatic cancer mice after intraperitoneal administration of an RNR gene-deficient virus.
Fig. 10 is a chart showing the survival rates of peritoneally metastasized human pancreatic cancer mice after intraperitoneal administration of an RNR gene-deficient virus.
Fig. 11 is a chart showing viral symptom scores of peritoneally metastasized human pancreatic cancer mice after intraperitoneal administration of an RNR gene-deficient virus.
Fig. 12 is a chart showing the survival rates of peritoneally metastasized human pancreatic cancer mice after intraperitoneal administration of an RNR gene-deficient virus.
Fig. 13 is a chart showing viral symptom scores of orthotopically transplanted human pancreatic cancer mice after intravenous administration of an RNR gene-deficient virus.
Fig. 14 is a chart showing the survival rates of orthotopically transplanted human pancreatic cancer mice after intravenous administration of an RNR gene-deficient virus.
Fig. 15 is a graph showing the tumor volume in subcutaneously transplanted human pancreatic cancer mice after intratumoral administration of an RNR gene-deficient virus.

### EMBODIMENTS OF THE INVENTION

The gene nomenclature used herein is those used for the vaccinia virus Copenhagen strain and is also used for homologous genes in other poxvirus families, unless otherwise specified.

The oncolytic virus of the present invention can be prepared as a recombinant vaccinia virus by modifying the vaccinia virus genome. Examples of the parent strain vaccinia virus include Copenhagen, Western Reserve, Lister, LC16mO, LC16m8, TianTan, or Wyeth.

Such a vaccinia virus genome can be modified using, for example, the genome of LC16m8 vaccinia virus, which is a smallpox vaccine strain that has been administered to the human body (Morikawa, S., et al., Journal of General Virology, 79 (18), 11873-11891, 2005). The attenuated strain LC16m8 has a reduced efficiency of virus infection and transmission due to a mutation in the B5R gene, which encodes one of the EEV components of its parent strain LC16mO. It is desirable that the vaccinia virus of the present invention, the replication capacity of which is restricted in normal cells, proliferates and spreads markedly in cancer cells and exhibits strong cytotoxicity. Thus, for the construction of the oncolytic vaccinia virus, LC16mO or a virus obtained by changing the B5R gene of the LC16m8 virus genome to the DNA sequence of the B5R region of the LC16mO virus may be used.

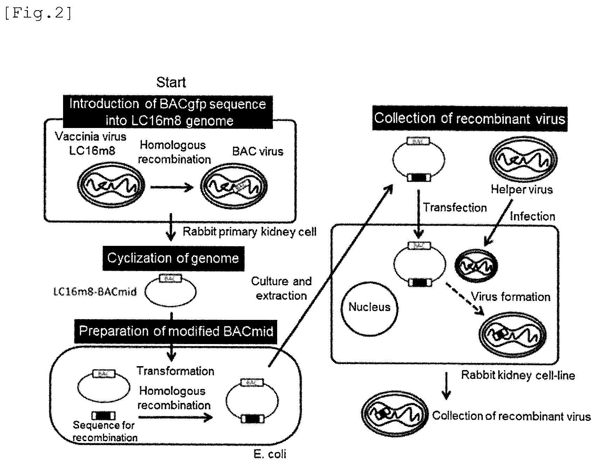

The vaccinia virus genome can be modified by homologous recombination using a vector. An isolated plasmid containing the modified DNA gene sequence is transfected into cultured cells with vaccinia virus infection. Recombination between the homologous viral DNA of the plasmid and the viral genome produces a modified virus due to the presence of the modified DNA sequence. A Bacterial Artificial Chromosome (BAC) system may be used to modify the vaccinia virus DNA. The BAC system involves a method in which the viral genome having the BACgfp sequence integrated is retained in Escherichia coli, and the viral genome is recombined by making use of genetics of Escherichia coli (Fig. 2).

The present invention makes it possible to obtain a growth-restricted vaccinia virus that can selectively replicate in proliferating cells and has improved safety by impairing the functions of VGF, ERK-activating protein, and RNR.

The gene encoding VGF is C11R, the gene encoding the O1L protein, which is an ERK-activating protein, is O1L, and the gene encoding RRM2, which is a small subunit of RNR, is F4L. By modifying these genes in the vaccinia virus genome, the functions of VGF, ERK activating protein, and RNR can be impaired.

Deficiency in the functions of VGF, O1L protein, and RRM2 in the vaccinia virus means that the C11R, O1L, and F4L genes are not expressed, or even if they are expressed, the expressed proteins fail to retain the normal functions of VGF, O1L protein, and RRM2. In order to impair the functions of VGF, O1L protein and RRM2, all or part(s) of the C11R, O1L, and F4L genes may be deleted. In addition, each gene may be mutated by nucleotide substitution, deletion, or addition so that neither normal VGF, O1L protein, nor RRM2 can be expressed. In addition, a foreign gene may be inserted into the C11R, O1L, or F4L gene. The foreign gene may be inserted, or the gene may be deleted or mutated by, for example, publicly-known homologous recombination or site-specific mutagenesis. In the present invention, when a normal gene product is not expressed due to a deletion or mutation of the gene, it can be said that the gene is deleted. Deficiency in the C11R, O1L, or F4L gene can be checked by PCR using a primer pair that specifically amplifies each gene.

Further, in addition to impairment of the functions of VGF, ERK-activating protein, and RNR, the gene(s) encoding the EEV-related protein(s) is replaced by the corresponding gene(s) of another vaccinia virus strain having a high EEV productivity. This can improve productivity of a restricted-proliferation vaccinia virus with improved safety.

The gene(s) encoding the EEV-related protein(s) to be modified may be at least one of the genes encoding the seven different proteins (A33R, A34R, A36R, A56R, B5R, F12L, and F13L) that are components of EEV. For example, the productivity of the vaccinia virus can be improved by modifying the gene encoding A34R. It is preferable to modify all of A33R, A36R, A56R, B5R, F12L, and F13L, and it is more preferable to modify all of A33R, A34R, A36R, A56R, B5R, F12L, and F13L.

Examples of the vaccinia virus from which the DNA sequence(s) used to modify the gene(s) encoding the EEV-related protein(s) is derived include, but are not limited to, IHD-J or IHD-W.

In the present invention, in order to improve productivity, a growth-restricted vaccinia virus of interest is obtained by replacing the gene(s) encoding the EEV-related protein(s) by the corresponding gene(s) of another vaccinia virus strain having a high EEV productivity. This vaccinia virus is not particularly limited as long as the growth-restricted vaccinia virus does not replicate in normal cells, and can selectively replicate in proliferating cells. In the present invention, the growth-restricted vaccinia virus is the vaccinia virus of the present invention, which has improved safety by impairing the functions of VGF, ERK-activating protein, and RNR. Even the virus lacking C11R and O1L (MD-RVV) can have improved productivity by replacing the gene(s) encoding the EEV-related protein(s) by the corresponding gene(s) of another vaccinia virus strain having a high EEV productivity.

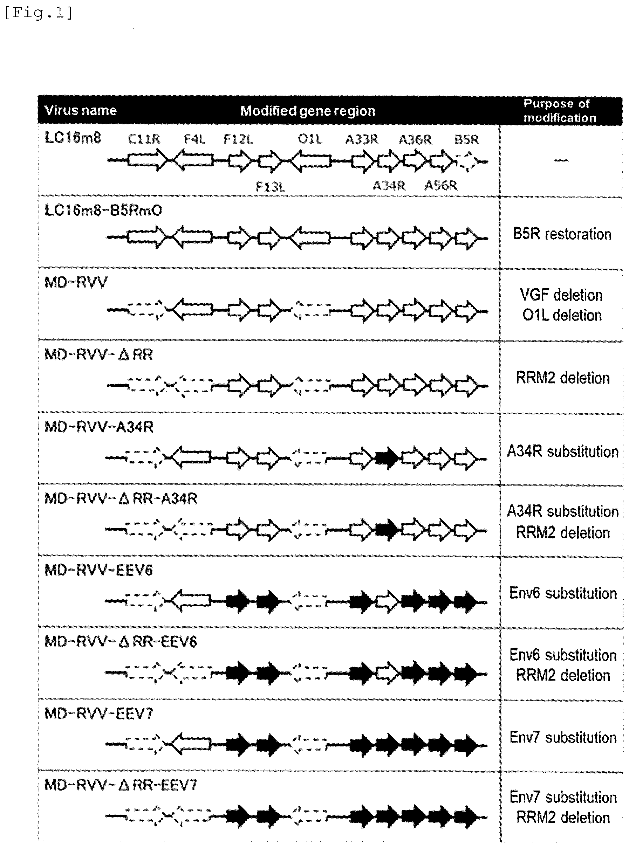

Fig. 1 illustrates the modified vaccinia viruses produced according to the present invention, and modified vaccinia viruses as described herein.

"LC16m8-B5RmO" is a virus in which the B5R gene of LC16m8 is modified to the DNA sequence of the B5R gene of its parent virus, LC16mO.

"MD-RVV" is a virus lacking the C11R and O1L genes of the LC16m8-B5RmO virus.

"MD-RVV-ΔRR" is a virus in which the F4L gene of MD-RVV virus is deleted.

"MD-RVV-A34R" is a virus in which the A34R gene of MD-RVV is replaced with the DNA sequence of the A34R gene of the IHD-J vaccinia virus strain.

"MD-RVV-ΔRR-A34R" is a virus in which the A34R gene of MD-RVV-ΔRR virus is replaced with the DNA sequence of the A34R gene of the IHD-J vaccinia virus strain.

"MD-RVV-EEV6" is a virus in which the A33R, A36R, A56R, B5R, F12L, and F13L genes of the MD-RVV virus are replaced with the DNA sequences of the A33R, A36R, A56R, B5R, F12L, and F13L genes of the IHD-J vaccinia virus strain, respectively.

"MD-RVV-ΔRR-EEV6" is a virus in which the A33R, A36R, A56R, B5R, F12L, and F13L genes of the MD-RW-ΔRR virus are replaced with the DNA sequences of the A33R, A36R, A56R, B5R, F12L, and F13L genes of the IHD-J vaccinia virus strain, respectively.

"MD-RVV-EEV7" is a virus in which the A33R, A34R, A36R, A56R, B5R, F12L, and F13L genes of the MD-RVV virus are replaced with the DNA sequences of the A33R, A34R, A36R, A56R, B5R, F12L, and F13L genes of the IHD-J vaccinia virus strain, respectively.

"MD-RVV-ΔRR-EEV7" is a virus in which the A33R, A34R, A36R, A56R, B5R, F12L, and F13L genes of the MD-RW-ΔRR virus are replaced by the DNA sequences of A33R, A34R, A36R, A56R, B5R, F12L, and F13L genes of the IHD-J vaccinia virus strain, respectively.

As used herein, the "proliferating cell(s)" means a cell(s) having a higher proliferative function than a normal cell(s), and examples thereof include, but are not limited to, a cancer cell(s) or a malignant tumor cell(s).

The growth-restricted vaccinia virus of the present invention can be used in both tumor cells and host cells used for industrial production. The tumor cells mainly mean malignant tumor cells and have the same meaning as cancer cells. Examples of the target cancer cell(s) when classified by an organ of origin include, but are not particularly limited to, a cancer cell(s) of any cancer type such as lung cancer, pancreatic cancer, ovarian cancer, skin cancer, gastric cancer, liver cancer, colon cancer, anal/rectal cancer, esophageal cancer, uterine cancer, breast cancer, bladder cancer, prostate cancer, esophageal cancer, brain/nerve tumor, lymphoma/leukemia, bone/osteosarcoma, smooth muscle myoma, or striated muscle myoma. Examples of the host cells used for industrial production include mammalian cells (e.g., Vero, GL37, CHO, HeLa, MRC-5, huGK-14) used for the production of vaccines or biomedicines.

The growth-restricted vaccinia virus of the present invention can be produced as a pharmaceutical composition by any pharmaceutical method for administering the virus into the body of a mammal including humans. For example, mammalian cells cultured in a bioreactor are used as a host to inoculate and culture the virus of the present invention. Then, the virus of interest is extracted from the cell culture medium, and purified. After that, for example, a pharmaceutically acceptable salt, is added to produce a preparation.

The pharmaceutical composition containing the growth-restricted vaccinia virus of the present invention comprises a pharmaceutically effective amount of the growth-restricted vaccinia virus of the present invention as an active ingredient, and may be in the form of a sterile aqueous or nonaqueous solution, a suspension, or an emulsion. The present invention provides a pharmaceutical composition for use in treating a cancer, comprising the vaccinia virus of the invention. Further, the pharmaceutical composition optionally comprises, for instance, a pharmaceutically acceptable diluent, aid, or carrier (e.g., a salt, a buffer, an adjuvant). The administration method is not particularly limited and the pharmaceutical composition can be administered in vivo using a method known to those skilled in the art. Examples include an intratumoral, intravenous, arterial, intraperitoneal, intracutaneous, subcutaneous, intramuscular, intraventricular, intrathoracic, intraspinal, intraepidermal, or mucosal surface injection. Preferred is intravenous, intraperitoneal, or intratumoral administration. Preferably, the pharmaceutical composition is administered systemically by intravenous administration. Systemic oncolytic virus therapy by intravenous administration can be conducted to treat not only primary tumors but also micrometastatic cancers. The effective dose may be determined, if appropriate, depending on the age, gender, health, body weight, etc., of each subject. The effective dose for human adults is not limited and is, for example, about 10⁶-10¹¹ plaque-forming units (PFU) and preferably 10⁸-10⁹ plaque-forming units (PFU) per dosing.

Cells infected with a vaccinia virus produce four viral forms that play distinct roles in the viral life cycle. IMV is the most abundant form of virus, suitable for mediating transmission between hosts due to its physically robust nature. However, IMV is not very suitable for the spread in a host due to its susceptibility to complements and antibodies. IEV acts as an intermediate between IMV and CEV/EEV, ensuring up-take of EEV-specific proteins, transporting virions to the cell surface using microtubules, and covering IMV particles with additional membranes and host proteins to decrease the susceptibility to antibodies and complements. This can widen the range of host receptors that can bind to the vaccinia virus. CEV is required to induce the formation of actin tails from the lower part of virions on the cell surface and promote efficient intercellular transport of the virus. Eventually, EEV is released from the cell surface and mediates the spread of infection in the host (Smith G. L., Journal of General Virology, 83 (Pt 12), 2915-2931, 2002). A virus preparation in the EEV form is desirable for the oncolytic vaccinia virus used for intravenous administration. However, since the morphology of virions changes continuously during the life cycle of the virus, not only EEV released into the culture supernatant during virus culture but also virions such as IMV leaked from cells destroyed by viral proliferation are mixed. From the viewpoint of productivity and physical stability, it is difficult to separate only EEV for production. Thus, modification of EEV-related molecule(s) can contribute to improved efficacy and productivity of the oncolytic virus preparation including various virion forms.

Fragments of cancer cells destroyed by oncolytic viruses are predicted to induce an antitumor immune response specific to autologous cancer. Various viral vectors incorporating genes for cancer-specific antigens and/or immune response regulators have been studied as cancer vaccines. The vaccinia virus has a relatively large viral genome allowing for insertion of the entire gene of the antigenic protein and has an ability to replicate in the cytoplasm rather than in the nucleus of the infected cell. Accordingly, the risk of integration of a genetic material into the genome of the host cell is minimized. Thus, the vaccinia virus is excellent as a viral vector. MVA strain of attenuated vaccinia virus, which has extremely low proliferation capacity in mammalian cells, has been used as a safe viral vector. Oncolytic viruses are attenuated viruses, the growth of which is significantly restricted in normal cells. Use of each oncolytic virus as a viral vector is advantageous in inducing an antitumor immune response (Guo, Z. S., et al., Journal for ImmunoTherapy of Cancer, 7 (1), 6, 2019). The oncolytic vaccinia virus of the present invention can also be used as a vector having a gene insertion. The inserted gene and the insertion site are not particularly limited. For example, there is a method of inserting a gene for a cancer-specific antigen, an immune response regulator, or a protein with affinity for a cancer cell surface antigen into or near the C11R, O1L, or F4L gene, the gene functions of which are to be impaired in the present invention, or into or near an EEV-related gene(s).

The oncolytic virus according to the present invention has improved safety and productivity by deleting C11R, O1L and F4L from the vaccinia virus genome and further substituting the amino acid sequences of a plurality of EEV-related proteins with the amino acid sequences of the IHD-J strain.

Next, the present invention will be described in more detail with reference to Examples. However, the present invention is not limited to these Examples.

### EXAMPLE 1

### Production of Growth-Restricted Vaccinia Virus by Modifying Genome of Vaccine Strain Vaccinia Virus

The genome of LC16m8 vaccinia virus, a smallpox vaccine strain that had been administered to the human body, (Morikawa, S., et al., Journal of General Virology, 79 (18), 11873-11891, 2005) was modified to produce recombinant vaccinia viruses (Fig. 1).

### 1. Construction of Construct LC16m8-BACmid

The vaccinia virus genome was modified using the BAC system (see Fig. 2). By homologous recombination in cell culture, a BAC virus in which a BACgfp sequence was inserted into the genome of the vaccinia virus LC16m8 strain was prepared, and the BAC virus genome was cyclized to construct LC16m8-BACmid. As the first step, an insertion plasmid (pUC-VVTK-BAC-EGFP) for introducing the BACgfp sequence as a marker into LC16m8 was constructed. The specific method included amplification of TK1 using TK1 primer Fw (SEQ ID **NO:** 1) and TK1 primer Re (SEQ ID NO: 2) while using the vaccinia virus LC16m8 strain genome (GenBank: AY678275.1) as a template, and amplification of TK2 using the strain genome as a template and TK2 primer Fw (SEQ ID **NO:** 3) and TK2 primer Re (SEQ ID **NO:** 4). The TK1 was digested with restriction enzymes KpnI and PacI and the TK2 was digested with restriction enzymes XbaI and PacI. Thereafter, each fragment of interest was purified from an agarose gel.

Next, a pUC119 plasmid (GenBank: U07650.1) was digested with restriction enzymes KpnI and XbaI, and purified. Then, the above two fragments were inserted into the KpnI/XbaI site of the alkaline phosphatase-treated plasmid to construct pUC119-TK1-2. Next, the pUCIDT-KAN-op7.5+EGFP plasmid (SEQ ID NO: 5) was artificially synthesized, digested with a restriction enzyme PacI, and purified. Subsequently, the pUC119-TK1-2 was digested with a restriction enzyme PacI, purified, and then ligated into the PacI site of the alkaline phosphatase-treated plasmid to construct pUC-VVTK-op7.5+EGFP plasmid. Further, a plasmid, in which the pBeloBAC11 sequence (GenBank: CVU51113.1) was inserted into a pBSII plasmid (GenBank: U25267.1), was digested with a restriction enzyme NotI and then purified from an agarose gel. The resulting plasmid was ligated into the NotI site of a plasmid prepared by digesting pUC-VVTK-op7.5+EGFP with a restriction enzyme NotI, purifying, and treating it with alkaline phosphatase. In this way, pUC-VVTK-BAC-EGFP plasmid was constructed.

Next, the following describes how to introduce the BACgfp sequence into the LC16m8 genome. Primary rabbit kidney cells (PRK) were infected with the vaccinia virus LC16m8 strain at MOI = 10, and then cultured for 1 h to recover the cells. The recovered cells were suspended in HeBS buffer, and the pUC-VVTK-BAC-EGFP plasmid linearized with a restriction enzyme HindIII was added and then electroporated. The electroporation solution was serially diluted, and PRK, which had been cultured on a 96-well plate, was infected therewith and cultured. Then, the expression of green fluorescent protein (GFP) was observed under a fluorescence microscope. Those having a fluorescence-positive plaque or cytopathic effect (CPE) in wells with a high dilution ratio were selected. The cells of each selected well were collected, sonicated, and centrifuged. Then, the supernatant was obtained as a virus (LC16m8-BACgfp) in which the BACgfp sequence was inserted into LC16m8.

Further, LC16m8-BACmid was constructed by cyclization of the LC16m8-BACgfp genome and cloning into Escherichia coli as follows. PRK infected with LC16m8-BACgfp at MOI = 5 was transfected with the pCAGGS-Cre plasmid (self-prepared) by using a transfection reagent. Subsequently, LC16m8-BACmid was extracted as a circularized viral genome, and then electroporated into Escherichia coli GS1783 (WO2014077096A1). The electroporation solution was cultured on a CG agar medium supplemented with chloramphenicol. After that, Escherichia coli carrying LC16m8-BACmid was selected using, as a marker, chloramphenicol resistance in the BACgfp sequence. Finally, each clone of interest was stocked in glycerol.

### 2. Construction of Modified BACmid

Each modified BACmid was prepared using LC16m8-BACmid as a template. Prepared in advance from cultured cells was a BACgfp-removal sequence cassette (SEQ ID NO: 6) subsequently required for recovering a recombinant virus from which the BACgfp sequence had been removed. How to prepare the BACgfp-removal sequence cassette was as follows. A pUC119 plasmid (GenBank: U07650.1) was digested with restriction enzymes HincII and BamHI, purified, and treated with alkaline phosphatase. Here, a pBSII plasmid (GenBank: U25267.1) was ligated with the pBeloBAC11 sequence (GenBank: CVU51113.1) to give a plasmid. This plasmid was digested with a restriction enzyme XbaI and purified to give a fragment. This fragment was digested with restriction enzymes ScaI and BglII, and purified. The resulting fragment was ligated with the above plasmid to construct pUC119-pBeloSB. Further, the pUC119-pBeloSB was digested with a restriction enzyme NruI and purified (pUC119-pBeloSB/NruI/elution). Next, by using the vaccinia virus LC16m8 strain genome (GenBank: AY678275.1) as a template, the TK region was amplified using TK primer Fw (SEQ ID NO: 7) and TK primer Re (SEQ ID NO: 8). In addition, by using pEPkan-S plasmid (Addgene) as a template, PCR was performed using kanamycin primer 1Fw (SEQ ID NO: 9) and kanamycin primer 1Re (SEQ ID NO: 10) to amplify the kanamycin resistance gene. The above two PCR products were purified. Three fragments including the pUC119-pBeloSB/NruI/elution were reacted using an In-Fusion HD Cloning Kit (Takara). Then, the reaction solution was introduced into Escherichia coli JM109 (Takara). After the reaction, the resulting bacteria were plated on a CG agar medium supplemented with ampicillin, kanamycin, and chloramphenicol to obtain a clone carrying the plasmid of interest (pUC119-BAC-SBTKdup). The pUC119-BAC-SBTKdup plasmid was extracted from the cultured Escherichia coli, digested with restriction enzymes PstI and KpnI, and purified to prepare pUC119-BAC-SBTKdup/PstI/KpnI/elution as a BACgfp-removal sequence cassette (SEQ ID NO: 6).

### B5R Modification Cassette:

In order to prepare a recombinant virus of interest, an expression cassette of a gene to be modified was prepared by the following protocol. Although the LC16m8 strain is an attenuated virus as a safe vaccine strain, it is desirable to use the LC16mO strain, which is a highly proliferative parent strain in cultured cells, in view of the efficacy of the oncolytic virus. LC16m8 is known to produce an incomplete B5R protein by frameshifting due to a single nucleotide deletion (guanine deletion) in the B5R gene sequence (Morikawa, S., et al., Journal of General Virology, 79(18), 11873-11891, 2005). Then, in order to construct a BACmid in which the B5R gene sequence of LC16m8-BACmid was changed to the sequence of LC16mO having a complete B5R gene sequence, a B5R modification cassette (SEQ ID NO: 11) was prepared by the following protocol. In the B5R gene sequence of LC16mO, about 1 kb each upstream or downstream from the above guanine, totaling 2132 bp, was artificially synthesized to construct pUCFk-B5RmO (SEQ ID NO: 12). This DNA was digested with a restriction enzyme EcoRI, purified, and then treated with alkaline phosphatase (pUCFk-B5RmO/EcoRI/elution/BAP). In addition, PCR was performed using the pEPkan-S plasmid (Addgene) as a template and kanamycin primer 2Fw (SEQ ID NO: 13) and kanamycin primer 2Re (SEQ ID NO: 14) to amplify the kanamycin resistance gene. The post-amplification product was purified, digested with a restriction enzyme EcoRI, and purified to prepare rKanI/EcoRI/elution. Then, pUCFk-B5RmO-rKanI was constructed by ligating the pUCFk-B5RmO/EcoRI/elution/BAP and the rKanI/EcoRI/elution. The pUCFk-B5RmO-rKanI was digested with restriction enzymes XbaI and DraI and then purified to prepare pUCFk-B5RmO-rKanI/XbaI/DraI/elution. This was used as a B5R modification cassette (SEQ ID NO: 11) for modification of the BACmid.

### C11R-Deficient Cassette:

A C11R-deficient cassette (SEQ ID NO: 15) was prepared in order to recover a virus lacking the VGF functions. A sequence lacking 255 bp from the start codon of the C11R gene sequence to the restriction enzyme AccI site and about 1 kb fragments before the sequence and after the site were artificially synthesized to construct pUC57-ΔVGF (SEQ ID NO: 16). The pUC57-ΔVGF was digested with a restriction enzyme AccI, purified, and treated with alkaline phosphatase to prepare pUC57-ΔVGF/AccI/elution/BAP. By using the pEPkan-S plasmid (Addgene) as a template, the kanamycin resistance gene was amplified by PCR using kanamycin primer 3Fw (SEQ ID NO: 17) and kanamycin primer 3Re (SEQ ID NO: 18), purified from an agarose gel, and then cloned into a TOPO vector (Invitrogen). The reaction solution was introduced into Escherichia coli JM109 (Takara), and a plasmid was extracted from the resulting colonies to produce TOPO-rKanI. The TOPO-rKanI was digested with a restriction enzyme AccI, purified, digested with a restriction enzyme ScaI, and purified from an agarose gel to prepare rKanI/AccI/ScaI/elution. Then, pUC57-ΔVGF-rKanI was constructed by ligating the pUC57-ΔVGF/AccI/elution/BAP and the rKanI/AccI/ScaI/elution. The pUC57-ΔVGF-rKanI was digested with a restriction enzyme ScaI, and further digested with restriction enzymes BamHI and EcoRI. The resulting fragment was purified from an agarose gel, and used as a C11R-deficient cassette (SEQ ID NO: 15) for modification of the BACmid.

### O1L-Deficient Cassette:

An O1L-deficient cassette (SEQ ID NO: 19) was prepared in order to recover a virus lacking the O1L functions. A 1049 bp sequence from the start codon of the O1L gene sequence to the restriction enzyme XbaI site was deleted. Next, a sequence in which the kanamycin resistance gene sequence (Addgene) was inserted at 50 bp immediately after the XbaI site was artificially synthesized. In this way, pUC57-ΔO1L-rKanI (SEQ ID NO: 20) was constructed. The pUC57-ΔO1L-rKanI was digested with restriction enzymes ScaI and EcoRI. The resulting fragment was purified from an agarose gel, and used as an O1L-deficient cassette (SEQ ID NO: 19) for modification of the BACmid.

### F4L-Deficient Cassette:

A F4L-deficient cassette (SEQ ID NO: 21) was prepared in order to recover a virus lacking the RNR functions. A 765 bp sequence from the start codon of the F4L gene sequence to the EcoRI site was deleted. Next, a sequence in which the kanamycin resistance gene sequence (Addgene) was inserted at 50 bp immediately after the EcoRI site was artificially synthesized. In this way, pUC57-ΔF4L-rKanI (SEQ ID NO: 22) was constructed. The pUC57-ΔF4L-rKanI was digested with restriction enzymes BamHI and HindIII. The resulting fragment was purified from an agarose gel, and used as an F4L-deficient cassette (SEQ ID NO: 21) for modification of the BACmid.

### Envelope Modification Cassettes:

To recover a virus obtained in which an EEV-related gene(s) was replaced by the DNA sequence(s) of IHD-J strain or IHD-W strain, each of A33R modification cassette (SEQ ID NO: 23), A34R modification cassette (SEQ ID NO: 24), A36R modification cassette (SEQ ID NO: 25), A33-34-36R modification cassette (SEQ ID NO: 26), A56R modification cassette (SEQ ID NO: 27), B5R modification cassette (SEQ ID NO: 28), or F12-13L modification cassette (SEQ ID NO: 29) was prepared. The specific method included: providing each gene sequence derived from IHD-J strain or IHD-W strain; artificially synthesizing a sequence in which the kanamycin resistance gene sequence (Addgene) was inserted at 50 bp immediately after a specific site; and constructing pUC57-A33R-rKanI (SEQ ID NO: 30), pUC57-A34R-rKanI (SEQ ID NO: 31), pUC57-A36R-rKanI (SEQ ID NO: 32), pUC57-A33-34-36R-rKanI (SEQ ID NO: 33), pUC57- A56R-rKanI (SEQ ID NO: 34), pUC57-B5R-rKanI (SEQ ID NO: 35), or pUC57-F12-13L-rKanI (SEQ ID NO: 36). The pUC57-B5R-rKanI (SEQ ID NO: 35) was digested with restriction enzymes XbaI and BglI. The other constructs were each digested with restriction enzymes BamHI and BglI. Each construct was purified from an agarose gel and used as an envelope modification cassette for modification of each BACmid.

### Construction of LC16m8-B5RmO-BACmid:

Construction of LC16m8-B5RmO-BACmid is described below. Escherichia coli carrying the above LC16m8-BACmid was cultured, and electroporated with the B5R modification cassette (SEQ ID NO: 11). The electroporation solution was cultured on CG agar medium supplemented with chloramphenicol and kanamycin. Each clone having the cassette introduced was selected by using kanamycin resistance in the cassette as a marker. Further, the kanamycin resistance gene was removed. Then, each clone of interest was obtained by using chloramphenicol resistance given in the BACgfp sequence. A gene fragment of interest was amplified by PCR using a primer pair (SEQ ID NO: 37 and SEQ ID NO: 38), and purified. Then, the nucleotide sequence was analyzed using a sequencing primer (SEQ ID NO: 37). This verified that the obtained clone was the desired modified BACmid (LC16m8-B5RmO-BACmid).

Other modified BACmids were also prepared by substantially the same protocol as above. The band size was checked by PCR using a primer pair specific to the corresponding modification. Each primer pair specific to the corresponding modification was as follows.
C11R deficiency check primer Fw (SEQ ID NO: 39) and C11R deficiency check primer Re (SEQ ID NO: 40);
O1L deficiency check primer Fw (SEQ ID NO: 41) and O1L deficiency check primer Re (SEQ ID NO: 42);
F4L deficiency check primer Fw (SEQ ID NO: 43) and F4L deficiency check primer Re (SEQ ID NO: 44);
A33R modification check primer Fw (SEQ ID NO: 45) and A33R modification check primer Re (SEQ ID NO: 46);
A34R modification check primer Fw (SEQ ID NO: 47) and A34R modification check primer Re (SEQ ID NO: 48);
A36R modification check primer Fw (SEQ ID NO: 49) and A36R modification check primer Re (SEQ ID NO: 50);
A56R modification check primer Fw (SEQ ID NO: 51) and A56R modification check primer Re (SEQ ID NO: 52);
B5R modification check primer Fw (SEQ ID NO: 53) and B5R modification check primer Re (SEQ ID NO: 54); and
F12-13L modification check primer Fw (SEQ ID NO: 55) and F12-13L modification check primer Re (SEQ ID NO: 56).

Further, after genome purification, each modification check sequencing primer [A33R modification (SEQ ID NO: 45), A34R modification (SEQ ID NO: 47), A36R modification (SEQ ID NO: 49), A56R modification (SEQ ID NO: 51, SEQ ID NO: 57), B5R modification (SEQ ID NO: 53), or F12-13L modification (SEQ ID NO: 55, SEQ ID NO: 58, SEQ ID NO: 59, SEQ ID NO: 60)] was used for nucleotide sequence analysis to confirm the obtained clone was the desired modified BACmid (MD-RVV-BACmid, MD-RVV-ΔRR-BACmid, MD-RVV-EEV7-BACmid, MD-RVV-EEV6-BACmid, MD-RVV-A34R-BACmid, MD-RW-ΔRR-EEV7-BACmid, MD-RVV-ΔRR -EEV6-BACmid, or MD-RW-ΔRR-A34R-BACmid) .

A BACgfp-removal sequence cassette (SBTKdup sequence) (SEQ ID NO: 6) was introduced into each modified BACmid in a similar manner. The band size was checked by PCR using a primer pair (BACgfp synthesis primer Fw (SEQ ID NO: 61) and BACgfp synthesis primer Re (SEQ ID NO: 62)). The PCR product was purified and the nucleotide sequence was analyzed using a sequencing primer (SEQ ID NO: 61). Each obtained clone was confirmed to be the desired modified BACmid (MD-RVV-SBTKdup-BACmid, MD-RVV-ΔRR-SBTKdup-BACmid, MD-RVV-EEV7-SBTKdup-BACmid, MD-RVV-EEV6-SBTKdup-BACmid, MD-RVV-A34R-SBTKdup-BACmid, MD-RVV-ΔRR-EEV7-SBTKdup-BACmid, MD-RVV-ΔRR-EEV6-SBTKdup-BACmid, or MD-RVV-ΔRR-A34R-SBTKdup-BACmid) .

### 3. Extraction of Viral Genome

Each BACmid-derived virus, which has a BACgfp-removal sequence cassette, is designed so that BACgfp can be removed while cultured cells are subcultured. Using the above-mentioned BAC virus (LC16m8-BACgfp) as a helper virus, a virus derived from LC16m8-B5RmO-SBTKdup-BACmid was recovered from RK13 cells. RK13 cells cultured on a 6-well plate were inoculated with LC16m8-BACgfp as a helper virus at MOI = 1. After culturing for 1 h, the virus was removed and a new culture medium was added. Next, the LC16m8-B5RmO-SBTKdup-BACmid and a transfection reagent were mixed. The mixture was reacted for 15 min, added to cells, and cultured overnight at 37°C. GFP expression was checked under a fluorescence microscope. Then, cells were harvested from a well having a high proportion of GFP fluorescence-negative plaques, frozen and thawed, sonicated, centrifuged to give the supernatant as a virus liquid. The virus liquid was serially diluted with culture medium, inoculated into RK13 cells cultured on a 96-well plate, and cultured at 37°C for two nights. Then, cells were harvested from a well having a high proportion of GFP fluorescence-negative plaques under a fluorescence microscope, frozen and thawed, sonicated, and centrifuged to give the supernatant as a virus liquid. This procedure was repeated, and virus purification was completed when all the viral plaques in the RK13 cells became fluorescently negative for two consecutive times. The recovered virus was inoculated into RK13 cells cultured on a 6-well plate, and cultured at 37°C for two nights. After that, the virus genome was extracted using a genome extraction kit. By using the extracted viral genome as a template, PCR was performed using a primer pair (SEQ ID NO: 61 and SEQ ID NO: 62) to verify, from the band size, removal of the BACgfp sequence. Further, the PCR product was purified, and the nucleotide sequence was analyzed using the BACgfp removal check primer (SEQ ID NO: 61) and the B5R guanine insertion check primer (SEQ ID NO: 37). This analysis verified removal of the sequence from the BACgfp sequence insertion site and further insertion of a nucleotide (guanine) at the specific site in the B5R gene sequence, which nucleotide is deleted in LC16m8. The resulting product was used as LC16m8-B5RmO.

Substantially the same protocol as above and LC16m8-BACgfp as a helper virus were used to produce a virus derived from MD-RVV-SBTKdup-BACmid, MD-RVV-ΔRR-SBTKdup-BACmid, MD-RVV-EEV7-SBTKdup-BACmid, MD-RVV-EEV6-SBTKdup-BACmid, MD-RVV-A34R-SBTKdup-BACmid, MD-RW-ΔRR-EEV7-SBTKdup-BACmid, MD-RVV-ΔRR-EEV6-SBTKdup-BACmid, or MD-RVV-ΔRR-A34R-SBTKdup-BACmid. PCR using the genome extracted therefrom as a template and a specific primer pair was performed to check the band size. Each specific primer pair was as follows.
C11R deficiency check primer Fw (SEQ ID NO: 39) and C11R deficiency check primer Re (SEQ ID NO: 40);
O1L deficiency check primer Fw (SEQ ID NO: 41) and O1L deficiency check primer Re (SEQ ID NO: 42);
F4L deficiency check primer Fw (SEQ ID NO: 43) and F4L deficiency check primer Re (SEQ ID NO: 44);
A33R modification check primer Fw (SEQ ID NO: 45) and A33R modification check primer Re (SEQ ID NO: 46);
A34R modification check primer Fw (SEQ ID NO: 47) and A34R modification check primer Re (SEQ ID NO: 48);
A36R modification check primer Fw (SEQ ID NO: 49) and A36R modification check primer Re (SEQ ID NO: 50);
A56R modification check primer Fw (SEQ ID NO: 51) and A56R modification check primer Re (SEQ ID NO: 52);
B5R modification check primer Fw (SEQ ID NO: 53) and B5R modification check primer Re (SEQ ID NO: 54); and
F12-13L modification check primer Fw (SEQ ID NO: 55) and F12-13L modification check primer Re (SEQ ID NO: 56); and
BACgfp synthesis primer Fw (SEQ ID NO: 61) and BACgfp synthesis primer Re (SEQ ID **NO:** 62).

Furthermore, each PCR product was purified and the nucleotide sequence was analyzed using each modification check sequencing primer [A33R modification (SEQ ID NO: 45), A34R modification (SEQ ID NO: 47), A36R modification (SEQ ID NO: 49), A56R modification (SEQ ID NO: 51, SEQ ID NO: 57), B5R modification (SEQ ID NO: 53), F12-13L modification (SEQ ID NO: 55, SEQ ID NO: 58, SEQ ID NO: 59, SEQ ID NO: 60), or BACgfp removal (SEQ ID NO: 61)]. This analysis verified that the obtained each clone was a desired recombinant virus (MD-RVV, MD-RVV-ΔRR, MD-RVV-EEV7, MD-RVV-EEV6, MD-RVV-A34R, MD-RVV-ΔRR-EEV7, MD-RVV-ΔRR-EEV6, or MD-RVV-ΔRR-A34R).

### EXAMPLE 2

### Decrease in Cytotoxicity in Normal Cells by RNR Gene Deficiency

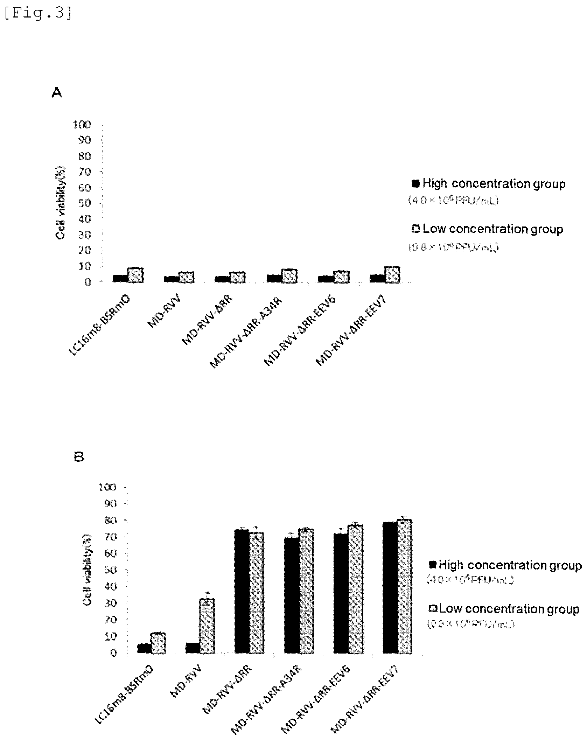

Cultured cells derived from cancer cells or normal cells were infected with MD-RVV, an oncolytic vaccinia virus lacking the C11R and O1L genes, or its EEV-related gene-modified viruses, or their modified viruses lacking the F4L gene encoding a small subunit of ribonucleotide reductase (RNR) and was present in their viral genomes. Then, the cytotoxicity was evaluated using a Cell Counting Kit-8 (DOJINDO LABORATORIES). Cancer cells (human cervical carcinoma-derived cell line: HeLa cells) and normal cells (normal human dermal fibroblasts: NHDF) were seeded on a 96-well plate and subjected to adhesion culture in a serum-containing medium. Next, the respective cells were cultured serum-starvedly in a serum-free medium and inoculated with a low level (0.8 × 10⁶ PFU/mL) or a high level (4.0 × 10⁶ PFU/mL) of each virus selected from LC16m8-B5RmO, MD-RVV, MD-RVV-ΔRR, MD-RVV-ΔRR-A34R, MD-RVV-ΔRR-EEV6, or MD-RVV-ΔRR-EEV7. After 72 h, a cell counting kit was used to quantify the cell viability of each virus inoculation group while the absorbance of the control group without virus inoculation was set to 100%. The results are shown in Fig. 3. All the viruses used here showed similar cytotoxicity to cancer cells (Fig. 3A), however the virus toxicity to the normal cells was significantly reduced in the F4L gene-deficient virus (ΔRR) (Fig. 3B).

### EXAMPLE 3

### Decrease in Virus Toxicity After Intravenous Administration to Immunodeficient Mice by RNR Gene Deficiency

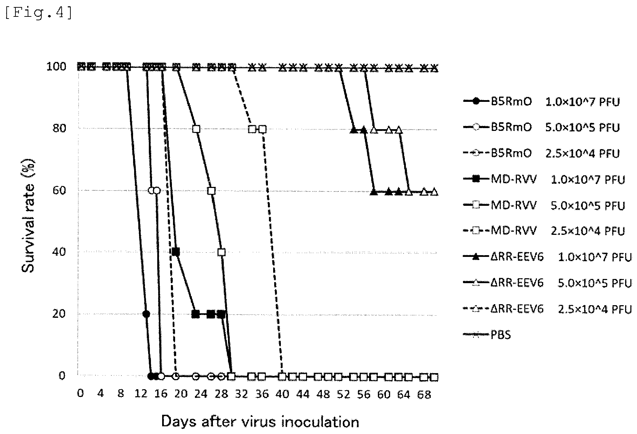

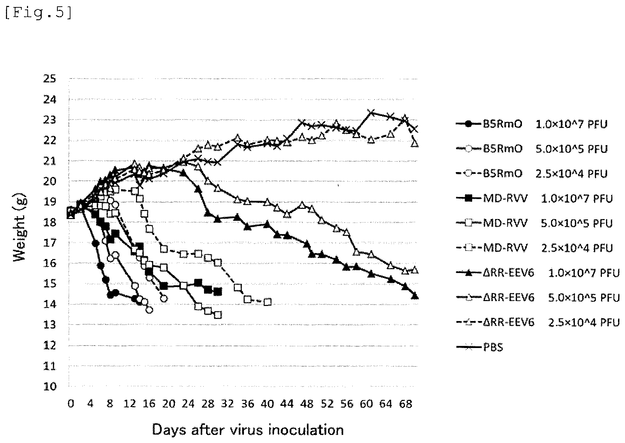

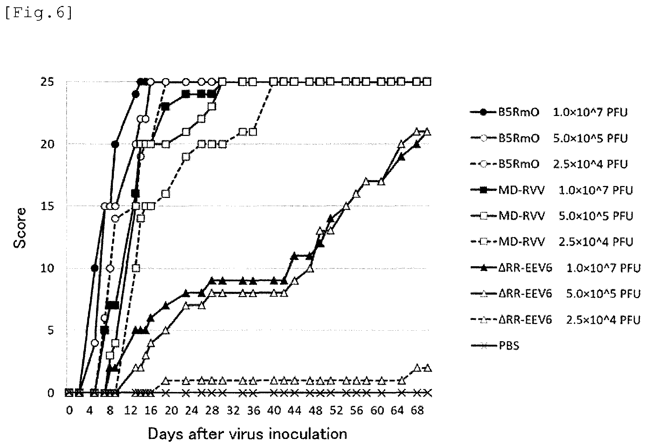

Immunodeficient mice (SCID mice, 5 weeks old, female, 5 in each group) were given LC16m8-B5RmO, MD-RVV, MD-RVV-ΔRR-EEV6 at 2.5 × 10⁴, 5 × 10⁵, or 1 × 10⁷ PFU/0.1 mL. The dose was intravenously administered to the mice, and the number of survivors, the body weight, and the viral symptom score were recorded. The viral symptom score is up to 5 points as follows: 0 points: no symptoms; 1 point: 1-2 smallpox lesions; 2 points: 3-4 smallpox lesions; 3 points: many smallpox lesions, a rough coat, piloerection; 4 Points: dyspnea, dying; and 5 points: death. The results are shown in Figs. 4 to 6. In the LC16m8-B5RmO and MD-RVV administration groups, animals in all dose groups died by 19 and 40 days after virus administration, respectively. However, for MD-RW-ΔRR-EEV6, 3 out of 5 animals in the high-dose and medium-dose groups survived until 71 days when the observation was completed. In addition, all animals were alive in the low-dose group (Fig. 4). It was observed that both viruses caused a weight loss in a dose-dependent manner. The degree of weight loss was greater in the order from MD-RVV-ΔRR-EEV6 to MD-RVV to LC16m8-B5RmO (smaller to greater) (Fig. 5). The viral symptom score also depended on the dose and became higher in the order from MD-RVV-ΔRR-EEV6 to MD-RVV to LC16m8-B5RmO (lower to higher) (Fig. 6).

### EXAMPLE 4

### Increase in Productivity in Cancer Cells by Modifying EEV-Related Protein(s)

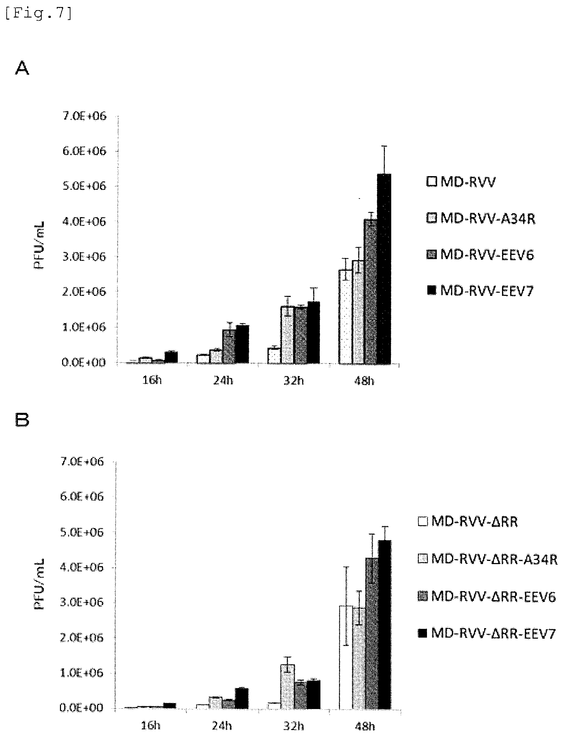

HeLa cells were seeded on a 24-well plate and subjected to adherent culture in a serum-containing culture medium. Next, the cells were inoculated with MD-RVV, MD-RVV-A34R, MD-RVV-EEV6, MD-RVV-EEV7, MD-RVV-ΔRR, MD-RVV-ΔRR-A34R, MD-RVV-ΔRR-EEV6, or MD-RVV-ΔRR-EEV7 at MOI = 1. After culturing for 1 h, the virus was removed and a fresh serum-containing culture medium was added. The culture medium was collected after 16, 24, 32, or 48 h to obtain each virus in the culture supernatant. The recovered virus was serially diluted, inoculated into RK13 cells cultured on a 6-well plate, and cultured for 1 h. Then, the virus was removed, and a methylcellulose medium was newly added. After culturing at 37°C for 72 h, plaques were counted to calculate the infectivity titer. The results are shown in Fig. 7. This demonstrated that, compared with an EEV-related protein(s) was modified in MD-RVV (Fig. 7A) or the virus lacking the F4L gene in MD-RVV (Fig. 7B), any of the modified viruses exhibited a tendency of increasing the amount of production of each virus in the culture supernatant throughout the culturing period. In particular, the greater the number of modifications of EEV-related proteins, the greater the effect.

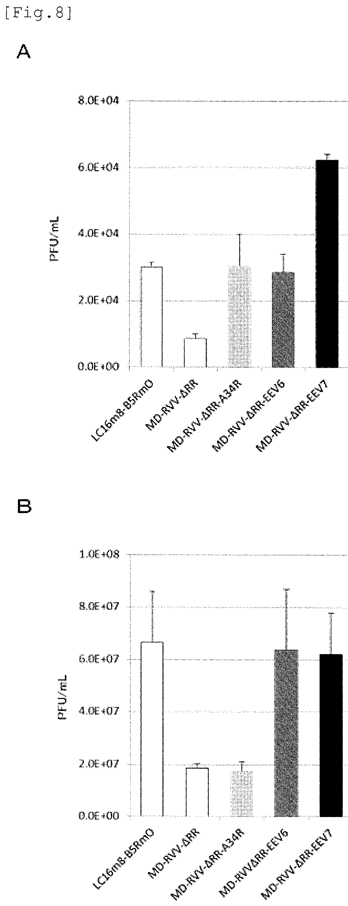

In addition, HeLa cells were seeded on a 24-well plate and subjected to adherent culture in a serum-containing culture medium. Next, the cells were cultured in a chemically defined culture medium and inoculated with LC16m8-B5RmO, MD-RVV-ΔRR, MD-RVV-ΔRR-A34R, MD-RVV-ΔRR-EEV6, or MD-RW-ΔRR-EEV7 at MOI = 0.01. After culturing for 1 h, the virus was removed and a fresh chemically defined culture medium was added. The culture medium was collected after 48 h to obtain each virus in the culture supernatant. Further, the cells were collected, frozen and thawed, sonicated, and centrifuged to obtain an intracellular virus in the supernatant. The recovered virus was serially diluted, inoculated into RK13 cells cultured on a 6-well plate, and cultured for 1 h. Then, the virus was removed, and a methylcellulose medium was newly added. After culturing at 37°C for 72 h, plaques were counted to calculate the infectivity titer. The results are shown in Fig. 8. Modification of the EEV-related protein(s) caused a tendency to regain the virus production decreased due to the deletion of the RNR gene in both the culture supernatant virus (Fig. 8A) and the intracellular virus (Fig. 8B). In particular, the greater the number of modifications of EEV-related proteins, the greater the effect.

### EXAMPLE 5

### Effects of RNR Gene-Deficient Virus Intraperitoneally Administered to Peritoneally Metastasized Human Pancreatic Cancer Mice

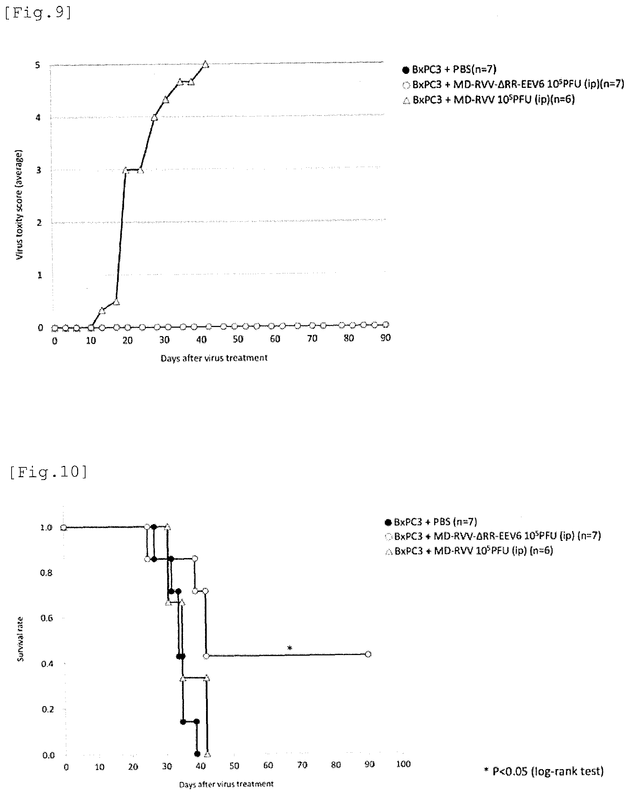

It was checked whether the RNR gene-deficient virus elicited a survival-prolonging effect on peritoneally metastasized model mice when administered intraperitoneally. For this purpose, peritoneally metastasized human pancreatic cancer mice were established by transplanting 3 × 10⁶ human pancreatic cancer BxPC3-Luc cells into the abdominal cavity of each immunodeficient mouse (SCID mice, 6 weeks old, female). Then, 21 days later, MD-RVV or MD-RVV-ΔRR-EEV6 (10⁵ PFU/0.1 mL) was intraperitoneally administered to the mice. In addition, PBS (0.1 mL) was intraperitoneally administered to the virus-non-administration group as a control the same 21 days later. After administration, the virus toxicity and the number of survivors were recorded. In addition, the virus toxicity was determined by scoring the symptoms with a maximum of 5 points as follows: 0 points: no symptoms; 1 point: 1-2 smallpox (pox) lesions; 2 points: 3-4 smallpox (pox) lesions; 3 points: many smallpox (pox) lesions, a rough coat, piloerection; 4 points: dyspnea, dying; and 5 points: virus-related death. The results are shown in Figs 9 to 10 (Fig. 9: viral symptom score; Fig. 10: survival rate) .

Regarding the virus toxicity, the MD-RVV-administration group manifested symptoms of smallpox (pox) from about 10 days after administration, and after that, severe viral symptoms such as an increased number of smallpox (pox) lesions, a rough coat, and dyspnea were developed. At last, by day 42, all cases died from their virus toxicity (Fig. 9). By contrast, the MD-RVV-ΔRR-EEV6 administration group showed no viral symptoms. This has demonstrated an effect of improving safety by the RNR gene deficiency (Fig. 9).

In the evaluation based on the number of survivors, the MD-RVV-administration group was accompanied by severe virus toxicity and all cases died at the same time as in the case of the virus-non-administration group in which the tumor grew (Fig. 10). By contrast, in the log-rank test for testing the difference in survival period, the MD-RVV-ΔRR-EEV6 administration group had a significant survival-prolonging effect when compared to the virus-non-administration group and the MD-RVV administration group (Fig. 10).

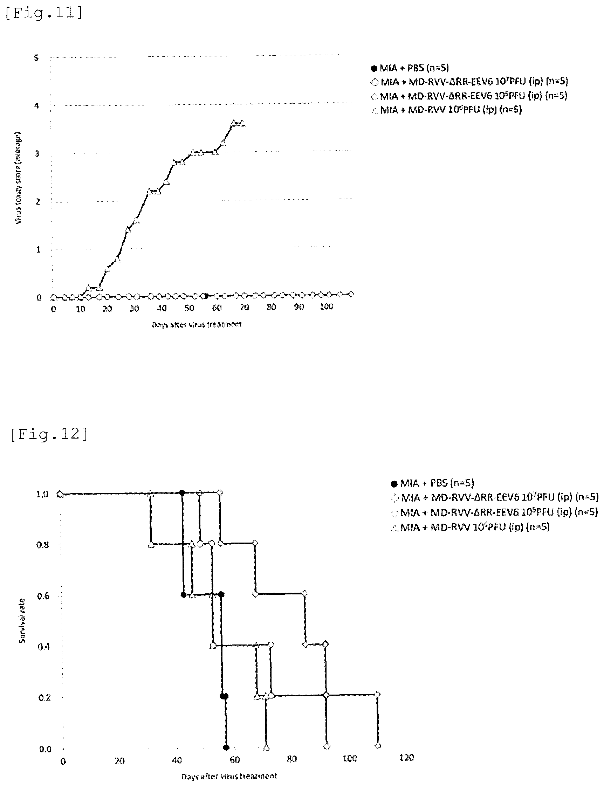

Subsequently, peritoneally metastasized human pancreatic cancer mice were established by transplanting different 3 × 10⁶ human pancreatic cancer MIA-PaCa2/CMV-Luc cells into the abdominal cavity of each immunodeficient mouse (SCID mice, 6 weeks old, female). Then, 14 days later, MD-RVV (10⁶ PFU/0.1 mL) or MD-RVV-ΔRR-EEV6 (10⁶ or 10⁷ PFU/0.1 mL) was intraperitoneally administered to the mice. In addition, PBS (0.1 mL) was intraperitoneally administered to the virus-non-administration group the same 14 days later. After administration, the virus toxicity and the number of survivors were recorded. In addition, the virus toxicity was determined by scoring the symptoms with a maximum of 5 points as follows: 0 points: no symptoms; 1 point: 1-2 smallpox (pox) lesions; 2 points: 3-4 smallpox (pox) lesions; 3 points: many smallpox (pox) lesions, a rough coat, piloerection; 4 points: dyspnea, dying; and 5 points: virus-related death. The results are shown in Figs. 11 and 12 (Fig. 11: viral symptom score, Fig. 12: survival rate).

Regarding the virus toxicity in the MD-RVV-administration group, a variation was observed between individuals with marked viral symptoms and individuals without them, however overall, moderate to severe viral symptoms were observed (Fig. 11). By contrast, the MD-RVV-ΔRR-EEV6 administration group showed no viral symptoms. This has demonstrated an effect of improving safety by the RNR gene deficiency (Fig. 11).

In the evaluation based on the number of survivors, all cases in the MD-RVV-administration group died at the same time as in the case of the non-virus-inoculation group due to severe virus toxicity and tumor growth (Fig. 12). By contrast, the MD-RW-ΔRR-EEV6-administration group showed a dose-dependent tendency to prolong survival when compared to the virus-non-administration group and the MD-RVV-administration group (Fig. 12).

The above results have suggested that the RNR gene-deficient virus is a highly safe oncolytic virus that can be administered intraperitoneally.

### EXAMPLE 6

### Effects of RNR Gene-Deficient Virus Intravenously Administered to Orthotopically Transplanted Human Pancreatic Cancer Mice

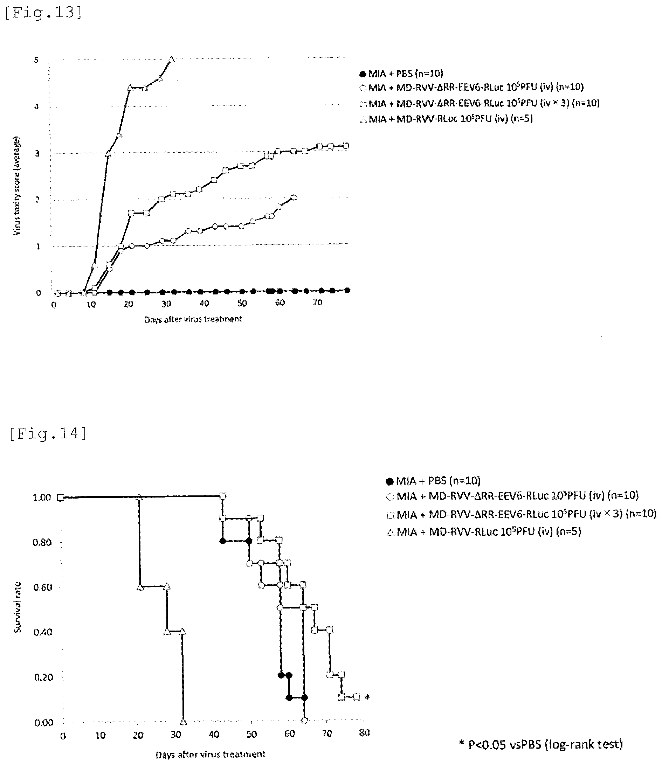

It was checked whether the RNR gene-deficient virus elicited a survival-prolonging effect on pancreatic cancer model mice when administered intravenously. For this purpose, orthotopically transplanted human pancreatic cancer mice were established by transplanting 1 × 10⁶ human pancreatic cancer MIA-PaCa2/CMV-Luc cells into the pancreatic membrane of each immunodeficient mouse (SCID mice, 5 weeks old, female). Then, 12 days later, MD-RVV-RLuc (10⁵ PFU/0.1 mL) or MD-RVV-ARR-EEV6-RLuc (10⁵ PFU/0.1 mL) was intravenously administered to the mice. Note that RLuc means a Renilla luciferase gene. To check the in vivo distribution of the virus in the future, a RLuc expression cassette (SEQ ID NO: 63) was constructed. Then, the cassette was inserted immediately before the C11R-AccI site after the deletion in Example 1. In addition, MD-RW-ΔRR-EEV6-RLuc was additionally administered twice within 1 week after the first administration (frequent administration). Further, PBS (0.1 mL) was intravenously administered to the virus-non-administration group the same 12 days later. After administration, the virus toxicity and the number of survivors were recorded. In addition, the virus toxicity was determined by scoring the symptoms with a maximum of 5 points as follows: 0 points: no symptoms; 1 point: 1-2 smallpox (pox) lesions; 2 points: 3-4 smallpox (pox) lesions; 3 points: many smallpox (pox) lesions, a rough coat, piloerection; 4 points: dyspnea, dying; and 5 points: virus-related death. The results are shown in Figs. 13 and 14 (Fig. 13: viral symptom score, Fig. 14: survival rate).

Regarding the virus toxicity, the MD-RVV-RLuc-administration group manifested symptoms of smallpox (pox) from about 10 days after administration, and after that, severe viral symptoms such as an increased number of smallpox (pox) lesions, a rough coat, and dyspnea were developed. At last, by day 32, all cases died from their virus toxicity accompanied (Fig. 13). By contrast, in the MD-RVV-ARR-EEV6-RLuc administration group, some viral symptoms were recognized after both single and frequent administrations, however no fatal symptoms were observed. An effect of improving safety by the RNR gene deficiency was demonstrated (Fig. 13).

In the evaluation based on the number of survivors, all cases in the MD-RVV-RLuc-administration group died of severe virus toxicity at a time earlier than in the case of the virus-non-administration group in which the tumor grew (Fig. 14). By contrast, in the log-rank test for testing the difference in survival period, the MD-RVV-ΔRR-EEV6-RLuc single administration group had substantially the same change in the survival as the virus-non-administration group. However, the frequent administration group elicited a more significant survival-prolonging effect than the virus-non-administration group (Fig. 14).

The above results have suggested that the RNR gene-deficient virus is a highly safe oncolytic virus that can be administered intravenously and frequently.

### EXAMPLE 7

### Effects of RNR Gene-Deficient Virus Intratumorally Administered to Subcutaneously Transplanted Human Pancreatic Cancer Mice

It was checked whether the RNR gene-deficient virus exerted an effect of suppressing tumor growth when administered intratumorally. For this purpose, 5 × 10⁶ human pancreatic cancer MIA-PaCa2/CMV-Luc cells were transplanted subcutaneously in the right femur of each immunodeficient mouse (SCID mice, 6 weeks old, female). The MD-RVV-ARR-EEV6-RLuc (10⁴, 10⁵, or 10⁶ PFU/0.1 mL) was intratumorally administered to individuals, the tumor volume of which reached 100 mm³ or larger. In addition, PBS (0.1 mL) was intratumorally administered to the virus-non-administration group. After administration, a change in the tumor volume was recorded. The tumor volume was calculated by minor axis × minor axis × major axis × 1/2.

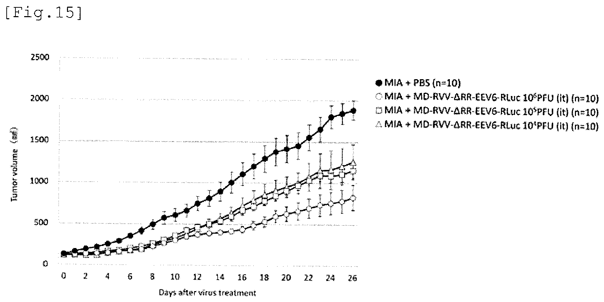

Regarding the change in the tumor volume, the MD-RVV-ΔRR-EEV6-RLuc showed a tendency to suppress the tumor growth in an approximately dose-dependent manner. An effect of suppressing the tumor growth after intratumoral administration was demonstrated (Fig. 15).

The results of Examples 5 to 7 have suggested that the RNR gene-deficient viruses MD-RW-ΔRR-EEV6 and MD-RVV-ΔRR-EEV6-RLuc exerted their intrinsic oncolytic effect in different human pancreatic cancer mice, thereby eliciting a survival-prolonging effect while the safety is secured.

### SEQUENCE LIST FREE TEXT

SEQ ID NO: 1 is the nucleotide sequence of TK1 primer Fw.
SEQ ID NO: 2 is the nucleotide sequence of TK1 primer Re.
SEQ ID NO: 3 is the nucleotide sequence of TK2 primer Fw.
SEQ ID NO: 4 is the nucleotide sequence of TK2 primer Re.
SEQ ID NO: 5 is the nucleotide sequence of the pUCIDT-KAN-op7.5+EGFP plasmid.
SEQ ID NO: 6 is the nucleotide sequence of the BACgfp-removal sequence cassette.
SEQ ID NO: 7 is the nucleotide sequence of TK primer Fw.
SEQ ID NO: 8 is the nucleotide sequence of TK primer Re.
SEQ ID NO: 9 is the nucleotide sequence of kanamycin primer 1Fw.
SEQ ID NO: 10 is the nucleotide sequence of kanamycin primer 1Re.
SEQ ID NO: 11 is the nucleotide sequence of the B5R modification cassette.
SEQ ID NO: 12 is the nucleotide sequence of pUCFk-B5RmO.
SEQ ID NO: 13 is the nucleotide sequence of kanamycin primer 2Fw.
SEQ ID NO: 14 is the nucleotide sequence of kanamycin primer 2Re.
SEQ ID NO: 15 is the nucleotide sequence of the C11R-deficient cassette.
SEQ ID NO: 16 is the nucleotide sequence of pUC57-ΔVGF.
SEQ ID NO: 17 is the nucleotide sequence of kanamycin primer 3Fw.
SEQ ID NO: 18 is the nucleotide sequence of kanamycin primer 3Re.
SEQ ID NO: 19 is the nucleotide sequence of the O1L-deficient cassette.
SEQ ID NO: 20 is the nucleotide sequence of pUC57-ΔO1L-rKanI.
SEQ ID NO: 21 is the nucleotide sequence of the F4L-deficient cassette.
SEQ ID NO: 22 is the nucleotide sequence of pUC57-ΔF4L-rKanI.
SEQ ID NO: 23 is the nucleotide sequence of the A33R modification cassette.
SEQ ID NO: 24 is the nucleotide sequence of the A34R modification cassette.
SEQ ID NO: 25 is the nucleotide sequence of the A36R modification cassette.
SEQ ID NO: 26 is the nucleotide sequence of the A33-34-36R modification cassette.
SEQ ID NO: 27 is the nucleotide sequence of the A56R modification cassette.
SEQ ID NO: 28 is the nucleotide sequence of the B5R modification cassette.
SEQ ID NO: 29 is the nucleotide sequence of the F12-13L modification cassette.
SEQ ID NO: 30 is the nucleotide sequence of pUC57-A33R-rKanI.
SEQ ID NO: 31 is the nucleotide sequence of pUC57-A34R-rKanI.
SEQ ID NO: 32 is the nucleotide sequence of pUC57-A36R-rKanI.
SEQ ID NO: 33 is the nucleotide sequence of pUC57-A33-34-36R-rKanI.
SEQ ID NO: 34 is the nucleotide sequence of pUC57-A56R-rKanI.
SEQ ID NO: 35 is the nucleotide sequence of pUC57-B5R-rKanI.
SEQ ID NO: 36 is the nucleotide sequence of pUC57-F12-13L-rKanI.
SEQ ID NO: 37 is the nucleotide sequence of the B5R modification check primer Fw.
SEQ ID NO: 38 is the nucleotide sequence of B5R modification check primer Re.
SEQ ID NO: 39 is the nucleotide sequence of the C11R deficiency check primer Fw.
SEQ ID NO: 40 is the nucleotide sequence of the C11R deficiency check primer Re.
SEQ ID NO: 41 is the nucleotide sequence of the O1L deficiency check primer Fw.
SEQ ID NO: 42 is the nucleotide sequence of the O1L deficiency check primer Re.
SEQ ID NO: 43 is the nucleotide sequence of the F4L deficiency check primer Fw.
SEQ ID NO: 44 represents the nucleotide sequence of the F4L deletion check primer Re.
SEQ ID NO: 45 represents the nucleotide sequence of the A33R modification check primer Fw.
SEQ ID NO: 46 represents the nucleotide sequence of the A33R modification check primer Re.
SEQ ID NO: 47 represents the nucleotide sequence of the A34R modification check primer Fw.
SEQ ID NO: 48 represents the nucleotide sequence of the A34R modification check primer Re.
SEQ ID NO: 49 represents the nucleotide sequence of the A36R modification check primer Fw.
SEQ ID NO: 50 represents the nucleotide sequence of the A36R modification check primer Re.
SEQ ID NO: 51 represents the nucleotide sequence of the A56R modification check primer Fw.
SEQ ID NO: 52 represents the nucleotide sequence of the A56R modification check primer Re.
SEQ ID NO: 53 represents the nucleotide sequence of the B5R modification check primer Fw.
SEQ ID NO: 54 represents the nucleotide sequence of the B5R modification check primer Re.
SEQ ID NO: 55 represents the nucleotide sequence of the F12-13L modification check primer Fw.
SEQ ID NO: 56 represents the nucleotide sequence of the F12-13L modification check primer Re.
SEQ ID NO: 57 represents the nucleotide sequence of the A56R modification check primer Fw.
SEQ ID NO: 58 represents the nucleotide sequence of the F12-13L modification check primer Fw.
SEQ ID NO: 59 represents the nucleotide sequence of the F12-13L modification check primer Fw.
SEQ ID NO: 60 represents the nucleotide sequence of the F12-13L modification check primer Fw.
SEQ ID NO: 61 represents the nucleotide sequence of the BACgfp synthesis primer Fw.
SEQ ID NO: 62 represents the nucleotide sequence of the BACgfp synthesis primer Re.
SEQ ID NO: 63 represents the nucleotide sequence of the RLuc expression cassette.

## Claims

1. A vaccinia virus deficient in functions of vaccinia virus growth factor (VGF), extracellular signal-regulated kinase (ERK)-activating protein, and ribonucleotide reductase (RNR), wherein all or part of regions of each of C11R, O1L, and F4L genes have been deleted or modified, and the functions of these gene products have been inactivated.

2. The virus according to claim 1, which is a growth-restricted virus having improved safety, wherein the virus does not replicate in a normal cell and can selectively replicate in a proliferating cell.

3. The virus according to claim 1, wherein the virus is a growth-restricted vaccinia virus, and a gene(s) encoding extracellular enveloped virus (EEV)-related protein(s) is replaced by a corresponding gene(s) of another vaccinia virus strain having a high EEV productivity, and wherein the virus does not replicate in a normal cell and can selectively replicate in a proliferating cell, thereby having improved safety and productivity.

4. The virus according to claim 3, wherein the gene(s) encoding the extracellular enveloped virus (EEV)-related protein(s)
(i) is one or more genes selected from the group consisting of A33R, A34R, A36R, A56R, B5R, F12L, and F13L, and has been replaced by a corresponding gene(s) of another vaccinia virus strain having a high EEV productivity;
(ii) are A33R, A36R, A56R, B5R, F12L, and F13L, and have been replaced by corresponding genes of another vaccinia virus strain having a high EEV productivity; or
(iii) are A33R, A34R, A36R, A56R, B5R, F12L, and F13L, and have been replaced by corresponding genes of another vaccinia virus strain having a high EEV productivity.

5. The virus according to claim 3 or 4, wherein another vaccinia virus strain having a high EEV productivity is IHD-J strain or IHD-W strain.

6. A pharmaceutical composition for use in treating a cancer, comprising the virus according to any one of claims 1 to 5.

7. The pharmaceutical composition for use according to claim 6, which is for intravenous administration, intraperitoneal administration, or intratumoral administration.

8. A growth-restricted vaccinia virus vector, which is the virus according to any one of claims 1 to 5 into which a foreign DNA has been introduced.

9. The vector according to claim 8, wherein the foreign DNA is a gene encoding a cancer-specific antigen, an immune response regulator, or a protein with affinity for a cancer cell surface antigen.

10. The virus according to claim 1, wherein the virus is a growth-restricted vaccinia virus having improved productivity, and a DNA sequence(s) of a gene(s) encoding extracellular enveloped virus (EEV)-related protein(s) of the growth-restricted vaccinia virus has been replaced by a DNA sequence(s) of a corresponding gene(s) of another vaccinia virus strain having a high EEV productivity, and wherein the virus does not replicate in a normal cell, and can selectively replicate in a proliferating cell.

11. The virus according to claim 10, wherein the gene(s) encoding the extracellular enveloped virus (EEV)-related protein(s)
(i) is one or more genes selected from the group consisting of A33R, A34R, A36R, A56R, B5R, F12L, and F13L;
(ii) are A33R, A36R, A56R, B5R, F12L, and F13L genes; or
(iii) are A33R, A34R, A36R, A56R, B5R, F12L, and F13L genes.

12. The virus according to claims 10 or 11, wherein another vaccinia virus strain having a high EEV productivity is IHD-J strain or IHD-W strain.

13. A pharmaceutical composition for use in treating a cancer, comprising the virus according to any one of claims 10 to 12.

14. A growth-restricted vaccinia virus vector, which is the virus according to any one of claims 10 to 12 into which a foreign DNA has been introduced.

15. The vector according to claim 14, wherein the foreign DNA is a gene encoding a cancer-specific antigen, an immune response regulator, or a protein with affinity for a cancer cell surface antigen.

## Patentansprüche

1. Vacciniavirus, dem die Funktionen des Vacciniavirus-Wachstumsfaktors (vaccinia virus growth factor; VGF), des Extracellular-signal Regulated Kinase (ERK)-aktivierenden Proteins und der Ribonucleotid-Reduktase (ribonucleotide reductase; RNR) fehlen, wobei alle oder ein Teil der Regionen jedes der Gene C11R, O1L und F4L deletiert oder modifiziert wurden und die Funktionen dieser Genprodukte inaktiviert wurden.

2. Virus nach Anspruch 1, das ein wachstumsrestriktiertes Virus ist, das verbesserte Sicherheit aufweist, wobei das Virus nicht in einer normalen Zelle repliziert und in einer proliferierenden Zelle selektiv replizieren kann.

3. Virus nach Anspruch 1, wobei das Virus ein wachstumsrestriktiertes Vacciniavirus ist und ein Gen/Gene, das/die ein Protein/Proteine codiert/codieren, das/die mit einem Extracellular Enveloped Virus (EEV) verwandt ist/sind, durch ein entsprechendes Gen/entsprechende Gene eines anderen Vacciniavirus-Stamms, der eine hohe EEV-Produktivität aufweist, ersetzt wurde/wurden, und wobei das Virus nicht in einer normalen Zelle repliziert und in einer proliferierenden Zelle selektiv replizieren kann, wodurch es eine verbesserte Sicherheit und Produktivität aufweist.

4. Virus nach Anspruch 3, wobei das Gen/die Gene, das/die das Protein/die Proteine codiert/codieren, das/die mit dem Extracellular Enveloped Virus (EEV) verwandt ist/sind
(i) ein oder mehrere Gene sind, die ausgewählt sind aus der Gruppe bestehend aus A33R, A34R, A36R, A56R, B5R, F12L und F13L und durch ein entsprechendes Gen/entsprechende Gene eines anderen Vacciniavirus-Stammes, der eine hohe EEV-Produktivität aufweist, ersetzt wurde/wurden;
(ii) A33R, A36R, A56R, B5R, F12L und F13L sind und durch entsprechende Gene eines anderen Vacciniavirus-Stammes, der eine hohe EEV-Produktivität aufweist, ersetzt wurden; oder
(iii) A33R, A34R, A36R, A56R, B5R, F12L und F13L sind und durch entsprechende Gene eines anderen Vacciniavirus-Stammes, der eine hohe EEV-Produktivität aufweist, ersetzt wurden.

5. Virus nach Anspruch 3 oder 4, wobei ein anderer Vacciniavirus-Stamm, der eine hohe EEV-Produktivität aufweist, der Stamm IHD-J oder der Stamm IHD-W ist.

6. Pharmazeutische Zusammensetzung zur Verwendung bei der Behandlung einer Krebserkrankung, umfassend das Virus nach einem der Ansprüche 1 bis 5.

7. Pharmazeutische Zusammensetzung zur Verwendung nach Anspruch 6, die zur intravenösen Verabreichung, intraperitonealen Verabreichung oder intratumoralen Verabreichung ist.

8. Wachstumsrestriktierter Vacciniavirus-Vektor, der das Virus nach einem der Ansprüche 1 bis 5 ist, in den eine fremde DNA eingebracht wurde.

9. Vektor nach Anspruch 8, wobei die fremde DNA ein Gen ist, das ein krebsspezifisches Antigen, einen Immunantwort-Regulator oder ein Protein mit Affinität zu einem Krebszell-Oberflächenantigen codiert.

10. Virus nach Anspruch 1, wobei das Virus ein wachstumsrestriktiertes Vacciniavirus ist, das eine verbesserte Produktivität aufweist, und eine DNA-Sequenz/DNA Sequenzen eines Gens/von Genen, das/die ein Protein/Proteine des wachstumsrestriktierten Vacciniavirus codiert/codieren, das/die mit einem Extracellular Enveloped Virus (EEV) verwandt ist/sind, durch eine DNA-Sequenz/DNA Sequenzen eines entsprechenden Gens/entsprechender Gene eines anderen Vacciniavirus-Stammes, der hohe EEV-Produktivität aufweist, ersetzt wurde/wurden, und wobei das Virus nicht in einer normalen Zelle repliziert und in einer proliferierenden Zelle selektiv replizieren kann.

11. Virus nach Anspruch 10, wobei das Gen/die Gene, das/die das Protein/die Proteine codiert/codieren, das/die mit dem Extracellular Enveloped Virus (EEV) verwandt ist/sind
(i) ein oder mehrere Gene sind, die ausgewählt sind aus der Gruppe bestehend aus A33R, A34R, A36R, A56R, B5R, F12L und F13L;
(ii) die Gene A33R, A36R, A56R, B5R, F12L und F13L sind; oder
(iii) die Gene A33R, A34R, A36R, A56R, B5R, F12L und F13L sind.

12. Virus nach den Ansprüchen 10 oder 11, wobei ein anderer Vacciniavirus-Stamm, der eine hohe EEV-Produktivität aufweist, der Stamm IHD-J oder der Stamm IHD-W ist.

13. Pharmazeutische Zusammensetzung zur Verwendung bei der Behandlung einer Krebserkrankung, umfassend das Virus nach einem der Ansprüche 10 bis 12.

14. Wachstumsrestriktierter Vacciniavirus-Vektor, der das Virus nach einem der Ansprüche 10 bis 12 ist, in den eine fremde DNA eingebracht wurde.

15. Vektor nach Anspruch 14, wobei die fremde DNA ein Gen ist, das ein krebsspezifisches Antigen, einen Immunantwort-Regulator oder ein Protein mit Affinität zu einem Krebszell-Oberflächenantigen codiert.

## Revendications

1. Virus de la vaccine déficient pour les fonctions du facteur de croissance du virus de la vaccine (VGF), de la protéine d'activation de kinase régulée par des signaux extracellulaires (ERK), et de la ribonucléotide réductase (RNR), dans lequel tout ou partie de régions de chacun parmi les gènes C11R, O1L et F4L a été supprimée ou modifiée, et les fonctions de ces produits géniques ont été inactivées.

2. Virus selon la revendication 1, qui est un virus à prolifération restreinte ayant une innocuité améliorée, lequel virus ne se réplique pas dans une cellule normale et peut se répliquer sélectivement dans une cellule proliférante.

3. Virus selon la revendication 1, lequel virus est un virus de la vaccine à prolifération restreinte, et un ou plusieurs gènes codant une ou plusieurs protéines associées au virus enveloppé extracellulaire (EEV) sont remplacés par un ou plusieurs gènes correspondants d'une autre souche de virus de la vaccine présentant une forte productivité d'EEV, et lequel virus ne se réplique pas dans une cellule normale et peut se répliquer sélectivement dans une cellule proliférante, en présentant ainsi une innocuité et une productivité améliorées.

4. Virus selon la revendication 3, dans lequel le ou les gènes codant une ou plusieurs protéines associées au virus enveloppé extracellulaire (EEV)
(i) sont un ou plusieurs gènes choisis dans le groupe constitué par A33R, A34R, A36R, A56R, B5R, F12L et F13L, et ont été remplacés par un ou plusieurs gènes correspondants d'une autre souche de virus de la vaccine présentant une forte productivité d'EEV;
(ii) sont A33R, A36R, A56R, B5R, F12L et F13L, et ont été remplacés par des gènes correspondants d'une autre souche de virus de la vaccine présentant une forte productivité d'EEV; ou
(iii) sont A33R, A34R, A36R, A56R, B5R, F12L et F13L, et ont été remplacés par des gènes correspondants d'une autre souche de virus de la vaccine présentant une forte productivité d'EEV.

5. Virus selon la revendication 3 ou 4, dans lequel une autre souche de virus de la vaccine présentant une forte productivité d'EEV est la souche IHD-J ou la souche IHD-W.

6. Composition pharmaceutique pour une utilisation dans le traitement d'un cancer, comprenant le virus de l'une quelconque des revendications 1 à 5.

7. Composition pharmaceutique pour une utilisation selon la revendication 6, qui est destinée à être administrée par voie intraveineuse, par voie intrapéritonéale, ou par voie intratumorale.

8. Vecteur viral de type virus de la vaccine à prolifération restreinte, qui est le virus de l'une quelconque des revendications 1 à 5 dans lequel un ADN étranger a été introduit.

9. Vecteur selon la revendication 8, dans lequel l'ADN étranger est un gène codant un antigène spécifique d'un cancer, un régulateur de réponse immunitaire, ou une protéine présentant une affinité pour un antigène de surface de cellule cancéreuse.

10. Virus selon la revendication 1, lequel virus est un virus de la vaccine à prolifération restreinte présentant une productivité améliorée, et dans lequel une ou plusieurs séquences d'ADN d'un ou plusieurs gènes codant une ou plusieurs protéines associées au virus enveloppé extracellulaire (EEV) du virus de la vaccine à prolifération restreinte ont été remplacées par une ou plusieurs séquences d'ADN d'un ou plusieurs gènes correspondants d'une autre souche de virus de la vaccine présentant une forte productivité d'EEV, et lequel virus ne se réplique pas dans une cellule normale, et peut se répliquer sélectivement dans une cellule proliférante.

11. Virus selon la revendication 10, dans lequel le ou les gènes codant une ou plusieurs protéines associées au virus enveloppé extracellulaire (EEV)
(i) sont un ou plusieurs gènes choisis dans le groupe constitué par A33R, A34R, A36R, A56R, B5R, F12L et F13L;
(ii) sont les gènes A33R, A36R, A56R, B5R, F12L et F13L; ou
(iii) sont les gènes A33R, A34R, A36R, A56R, B5R, F12L et F13L.

12. Virus selon la revendication 10 ou 11, dans lequel une autre souche de virus de la vaccine présentant une forte productivité d'EEV est la souche IHD-J ou la souche IHD-W.

13. Composition pharmaceutique pour une utilisation dans le traitement d'un cancer, comprenant le virus de l'une quelconque des revendications 10 à 12.

14. Vecteur du virus de la vaccine à prolifération restreinte, qui est le virus de l'une quelconque des revendications 10 à 12 dans lequel un ADN étranger a été introduit.

15. Vecteur selon la revendication 14, dans lequel l'ADN étranger est un gène codant un antigène spécifique d'un cancer, un régulateur de réponse immunitaire, ou une protéine présentant une affinité pour un antigène de surface de cellule cancéreuse.
